(19) Europäisches Patentamt
European Patent Office
Office européen des brevets

(11) EP 3 697 830 B1

(12) **EUROPÄISCHE PATENTSCHRIFT**

(45) Veröffentlichungstag und Bekanntmachung des Hinweises auf die Patenterteilung:
**13.12.2023 Patentblatt 2023/50**

(21) Anmeldenummer: **18783519.4**

(22) Anmeldetag: **16.10.2018**

(51) Internationale Patentklassifikation (IPC):
***C08G 65/40*** *(2006.01)* ***C08G 65/48*** *(2006.01)*

(52) Gemeinsame Patentklassifikation (CPC):
**C08G 65/4037; C08G 65/4006;** Y02E 60/50

(86) Internationale Anmeldenummer:
**PCT/EP2018/078184**

(87) Internationale Veröffentlichungsnummer:
**WO 2019/076860 (25.04.2019 Gazette 2019/17)**

(54) **POLYMERE ANIONEN LEITENDE MEMBRANE**
POLYMERIC ANIONS CONDUCTING MEMBRANES
MEMBRANES CONDUCTRICES D’ANIONS POLYMÈRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.10.2017 EP 17196802**

(43) Veröffentlichungstag der Anmeldung:
**26.08.2020 Patentblatt 2020/35**

(73) Patentinhaber: **Evonik Operations GmbH**
**45128 Essen (DE)**

(72) Erfinder:
- **CONRADI, Oliver**
**40545 Düsseldorf (DE)**
- **MALJUSCH, Artjom**
**44866 Bochum (DE)**
- **RÖGL, Harald**
**4702 Wallern an der Trattnach (AT)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**WO-A1-2017/117678**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

**Beschreibung**

Gegenstand der vorliegenden Erfindung sind Verbindungen, insbesondere polymere Verbindungen, die mindestens eine Imidazol- und/oder Imidazolium-Struktureinheit aufweisen, ein Verfahren zu deren Herstellung und deren Verwendung, insbesondere als Anionen leitende Membranen.

Polymere ionenleitende Membranen sind seit längerem bekannt. Die in WO 2005/045978 A2, US 2009325030 A1 und US 20040121210 A1 beschriebenen Membrane basieren auf einem hochfluorierten Polymerrückgrat.

In EP 2224523 B1 und US 20140014519 A1 werden Anionen leitende Membranen hergestellt, in dem ein poröser Film mit einer Mischung von verschiedenen Vinylgruppen aufweisenden Monomeren, von denen mindestens eines eine Halogen-Gruppe (Chlor-Gruppe) aufweist, getränkt wird, die Oberflächen des poröse Films jeweils mit einem Polyesterfilm abgedeckt werden und anschließend eine thermische Polymerisation durchgeführt wird. Das so erhaltene Material wird anschließend mit Trimethylamin oder Methyliodid und dann mit NaOH behandelt. In EP 2296210 A1 folgt auf die Behandlung mit Trimethylamin eine Behandlung mit $Na_2CO_3$.

In EP 2606954 A1 werden Anionen leitende Membrane durch die Härtung einer Polymerlösung erhalten, die Polymere enthält, die durch Chlormethylierung von Polysulfonen und anschließende Behandlung mit Trimethylamin erhalten wurden.

Aus dem Stand der Technik sind außerdem diverse Polymere bekannt, in denen Moleküle und/oder Molekülbausteine enthalten sind, die den nachfolgenden Formeln (Ia) und (Ib) entsprechen.

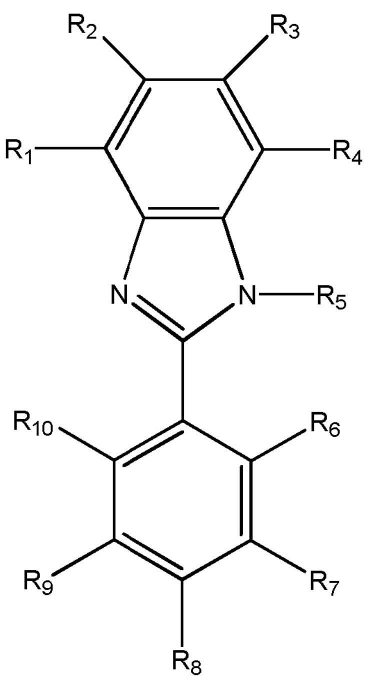

(Ia)

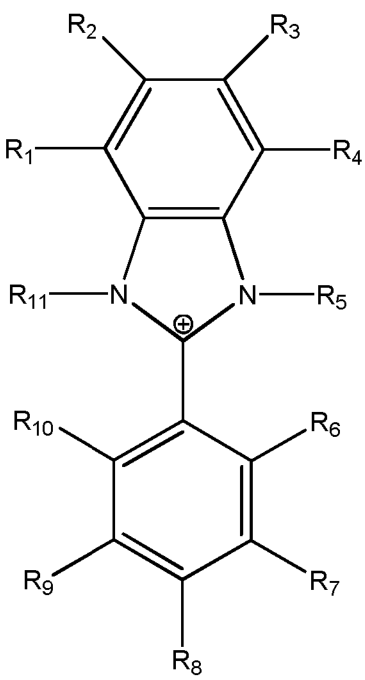

(Ib)

WO 2013/149328 beschreibt Polymere in denen Bausteine der Formel (Ib) enthalten sind, bei denen $R_1$, $R_2$, $R_3$, und $R_4$ gleich oder verschieden -H, eine beliebige Gruppe oder ein Polymerrest,

$R_5$ und $R_{11}$ gleich oder verschieden Methyl-, Trifluormethyl-, Alkyl-, Perfluoralkyl-, Heteroalkyl-, Aryl-, Aralkyl- oder ein Polmyerrest oder keine Gruppe,

$R_6$ und $R_{10}$ gleich oder verschieden Methyl-, Trifluormethyl-, Alkyl-, Perfluoralkyl-, Heteroalkyl-, Alkoxy-, Perfluoralkoxy-, Halogen-, Aryl-, Heteroaryl- oder ein Polymerrest, und

$R_7$, $R_8$ und $R_9$ gleich oder verschieden -H, beliebige Gruppe oder ein Polymerrest sind.

Als besondere Ausführungsformen werden solche Polymere beschrieben, bei denen die Einbindung des Bausteins (Ib) in das Polymer über die Reste $R_3$ und $R_8$, über den Rest $R_8$, über den Rest $R_5$ oder über die Reste $R_5$ und $R_{11}$, erfolgt. In den Beispielen werden als Bausteine (Ia) bzw. (Ib) 2-Phenylbenzimidazol, 2-Mesitylbenzimidazol, 1,3-Dimethyl-2-Mesitylbenzimidazolium, 1,3-Dimethyl-2-Phenylbenzimidazolium, Poly-(2,2'-(m-Phenyl)-5,5'-dibenzimidazol), Poly-(2,2'-(m-Phenyl)-5,5'-bis-(N,N'-Dimethylbenzimidazolium) Iodide), Poly-(2,2'-(m-Mesityl)-5,5'-dibenzimidazol) und Poly-(2,2'-(m-Mesityl)-5,5'-bis-(N,N'-Dimethylbenzimidazolium) Iodide) beschrieben.

In EP 0126231 B1 werden Moleküle bzw. Polymere beschrieben, in denen Bausteine der Formel (Ia) oder (Ib) enthalten sind, bei denen

$R_1$, $R_2$, $R_3$, und $R_4$ = -H,
$R_5$ und $R_{11}$ gleich oder verschieden H-, Kohlenwasserstoffrest mit 1 bis 10 C-Atomen oder Carboxyalkylrest mit 2 bis 10 C-Atomen, vorzugsweise Methyl-, Ethyl-, Benzyl- oder Carboxymethylrest,
$R_8$ = Vinyl-Rest oder ein Polymerrest, und
$R_6$, $R_7$, $R_9$ und $R_{10}$ gleich oder verschieden -H oder Kohlenwasserstoffgruppe mit 1 bis 4 Kohlenstoffatomen, vorzugsweise -H, Methyl- oder Ethylrest, sind.

In US 3817749 werden Moleküle gemäß Formel (Ia) beschrieben mit $R_1$, $R_2$, $R_3$, $R_4$, $R_5$, $R_7$, $R_8$ und $R_9$ = -H und $R_6$ und $R_{10}$ gleich Cl oder Br. Die mit Acetylen umgesetzten und anschließend polymerisierten Moleküle werden als photosensitive Polymere verwendet.

In WO 2017/117678 sowie den entsprechenden Publikationen von Steven Holdcroft werden Polymere und deren Verwendung in Ionen-leitenden Membranen beschrieben, die Einheiten der Formel (Ib), mit R10 und R6 = Phenylreste, aufweisen. Als Polymere werden Poly(4,4"-[2'-(1-methyl-1*H*-benzimidazol-2-yl)-*m*-terphenylene]) und Poly(4,4"-[2'-(1,3-dimethyl-1*H*-benzimidazolium-2-yl)-m-terphenylene])iodid beschrieben.

Aufgabe der vorliegenden Erfindung war die Bereitstellung von alternativen Verbindungen die als oder zur Herstellung von Anionen leitenden Polymeren geeignet sind.

Überraschenderweise wurde gefunden, dass diese Aufgabe von den anspruchsgemäßen Verbindungen gelöst wird.

Gegenstand der vorliegenden Erfindung sind deshalb Verbindungen wie in den Ansprüchen beansprucht und nachfolgend beschrieben.

Ebenfalls Gegenstand der vorliegenden Erfindung ist ein Verfahren zur Herstellung von solchen Verbindungen sowie deren Verwendung als Anionen leitende Membranen sowie diese Membranen selbst.

Die erfindungsgemäßen Polymeren haben den Vorteil, dass sie auf einfache Weise hergestellt werden können.

Die aus ihnen hergestellten Membranen haben den Vorteil, dass diese eine hohe mechanische Stabilität, ein geringes Quellverhalten kombiniert mit einer hohen dimensionalen Stabilität aufweisen. Zusätzlich dazu zeigen die Membrane recht hohe Anionenleitfähigkeiten.

Die erfindungsgemäßen Verbindungen, Verfahren und Verwendungen werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Werden nachfolgend Angaben in Prozent gemacht, so handelt es sich, wenn nicht anders angegeben um Angaben in Gewichts-%. Werden nachfolgend Mittelwerte angegeben, so handelt es sich, wenn nicht anders angegeben, um das Zahlenmittel. Werden nachfolgend Stoffeigenschaften, wie z. B. Viskositäten oder ähnliches angegeben, so handelt es sich, wenn nicht anders angegeben, um die Stoffeigenschaften bei 25 °C. Werden in der vorliegenden Erfindung chemische (Summen-)Formeln verwendet, so können die angegebenen Indizes sowohl absolute Zahlen als auch Mittelwerte darstellen.

Gegenstand der vorliegenden Erfindung sind Verbindungen, vorzugsweise Oligomere oder Polymere, bevorzugt Polymere, enthaltend mindestens einen Baustein der Formel (Ia) oder (Ib)

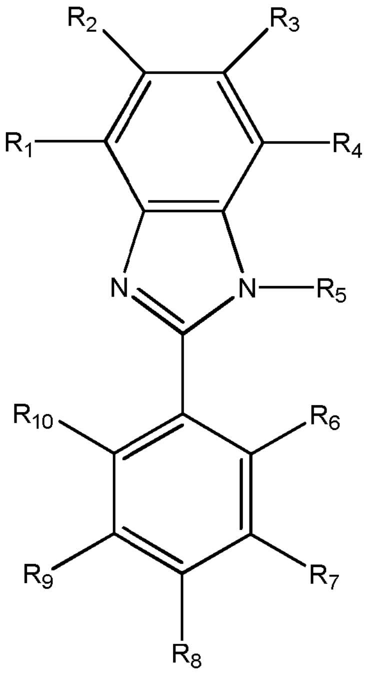

(Ia)

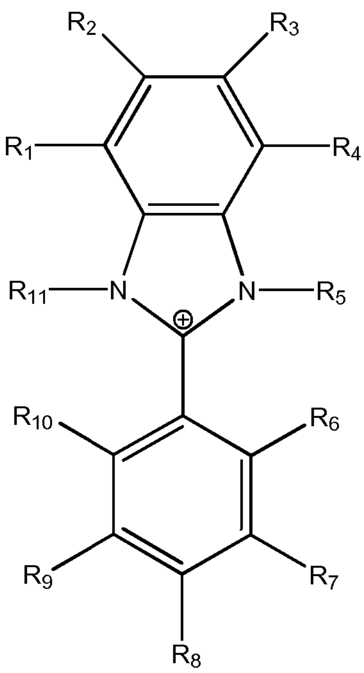

(Ib)

welche dadurch gekennzeichnet sind, dass $R_1$, $R_2$, $R_3$, und $R_4$ gleich oder verschieden -H, oder eine beliebige Gruppe, vorzugsweise gleich -H,

$R_5$ und $R_{11}$ gleich oder verschieden Alkyl- oder Perfluoralkyl-, vorzugsweise Methyl-, Ethyl-, Propyl-, iso-Propyl, n-Butyl-, tert-Butyl- oder Trifluormethylrest, bevorzugt gleich Methylrest,

$R_6$ und $R_{10}$ gleich oder verschieden Oligomer- oder Polymerrest; $R_6$ und $R_{10}$ sind vorzugsweise ein Polymerrest, und $R_7$, $R_8$ und $R_9$ sind gleich oder verschieden -H oder eine beliebige Gruppe, vorzugsweise gleich -H.

Erfindungsgemäße Polymere weisen mindestens einen Baustein der Formel (Ia) oder (Ib) wie oben definiert auf, wobei mindestens einer der Reste $R_6$ oder $R_{10}$ ein Oligomer- oder Polymerrest ist, der über ein Sauerstoffatom an das Ringkohlenstoffatom angebunden ist ($R_6$ oder $R_{10}$ = $-OR_{16}$ mit $R_{16}$ = Oligomer- oder Polymerrest, vorzugsweise ein Polymerrest).

Vorzugsweise sind die erfindungsgemäßen Verbindungen Oligomere oder Polymere, welche mindestens 2 Bausteine der Formel (Ia) oder (Ib), vorzugsweise (Ia) aufweisen. Besonders bevorzugte erfindungsgemäße Verbindungen sind Blockcopolymere.

Bei den erfindungsgemäßen Verbindungen handelt es sich vorzugsweise um Polymerverbindungen, deren Polymerreste $R_6$ und $R_{10}$ einen oder mehrere der Bausteine der Formel (IIa) und/oder (IIb) und/oder (IIIa) und/oder (IIIb) und/oder (IIIc) und/oder (IIId)

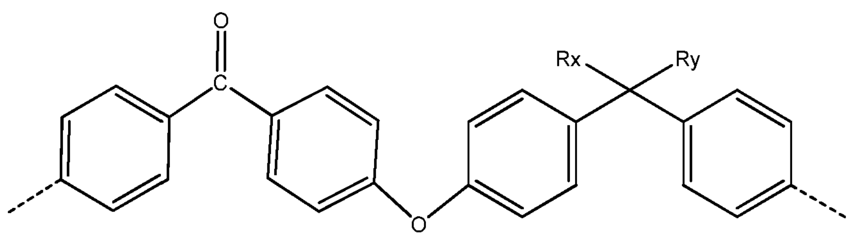

(IIa)

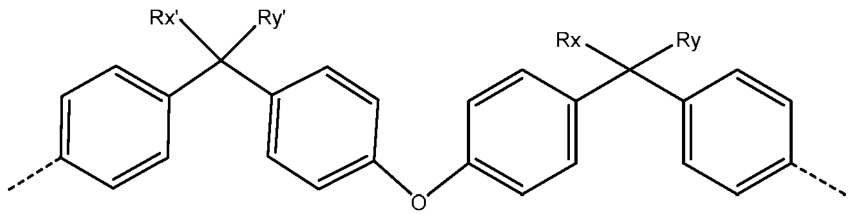

(IIb)

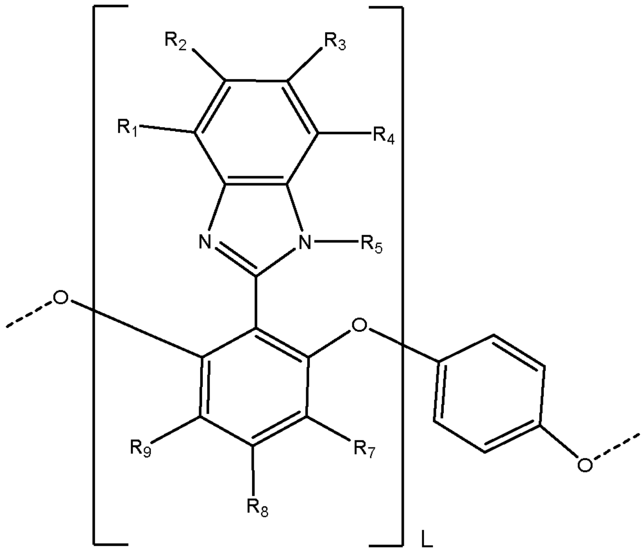

(IIIa)

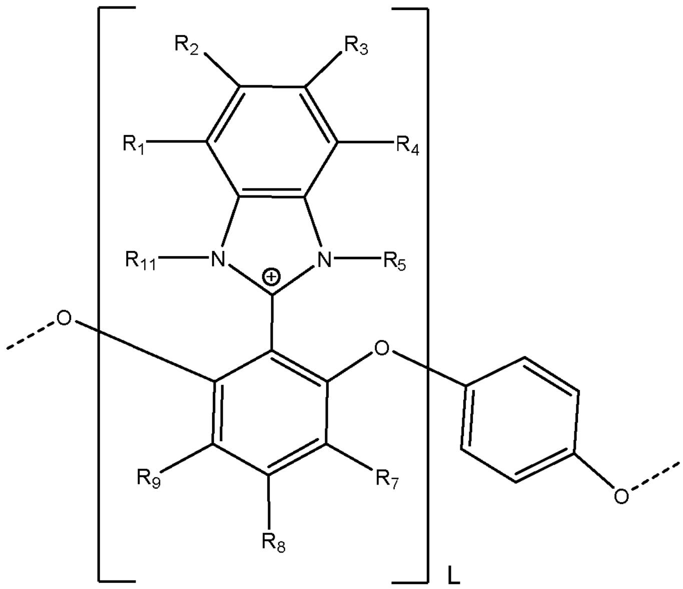

(IIIb)

.

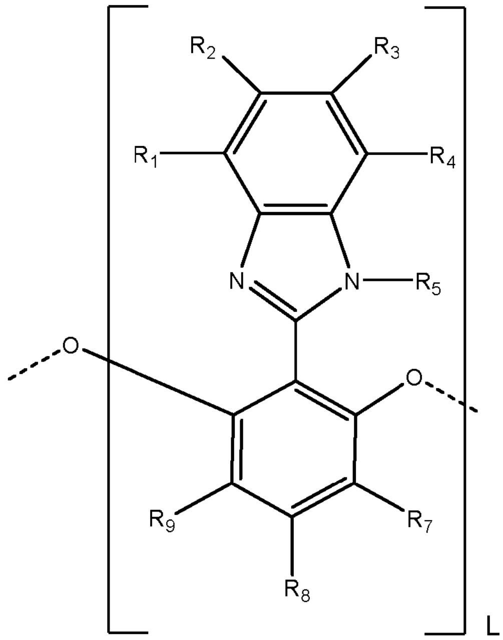

(IIIc)

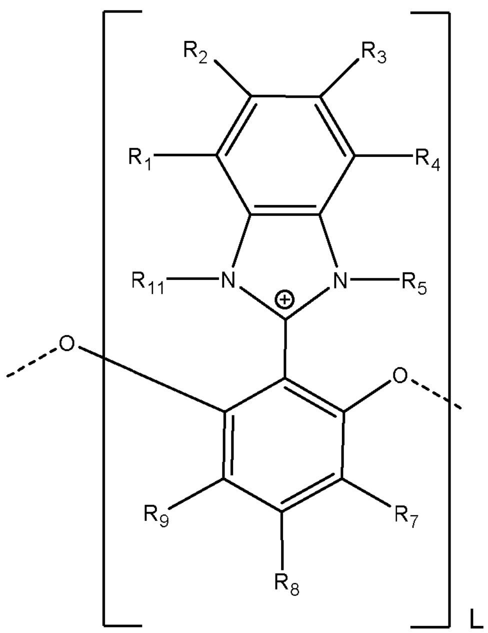

(IIId)

aufweisen, mit $R_1$, $R_2$, $R_3$, $R_4$, $R_5$, $R_7$, $R_8$, $R_9$ und $R_{11}$ wie vorstehend definiert, L = 1 bis 25, vorzugsweise 1 bis 15, bevorzugt 1 bis 9, und Rx und Ry und Rx' und Ry' gleich oder verschieden Alkyl-, Phenyl- oder Perfluoralkylrest, bevorzugt $-CH_3$ oder $-CF_3$.

Bevorzugte erfindungsgemäße Verbindungen sind Verbindungen der Formel (IVa), (IVb), (IVc), (IVd), (IVe) oder (IVf)

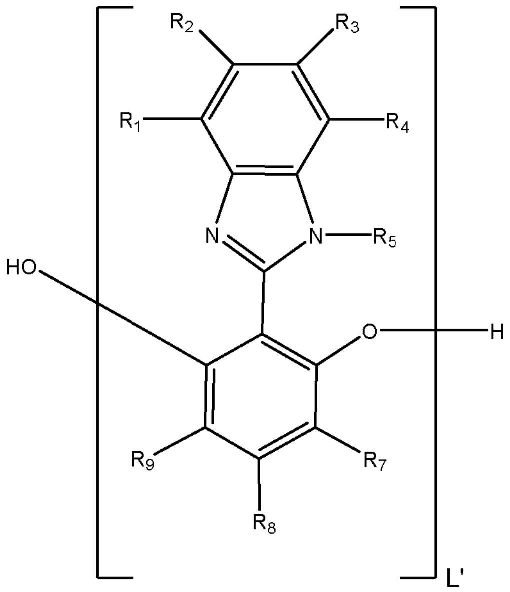

(IVa)

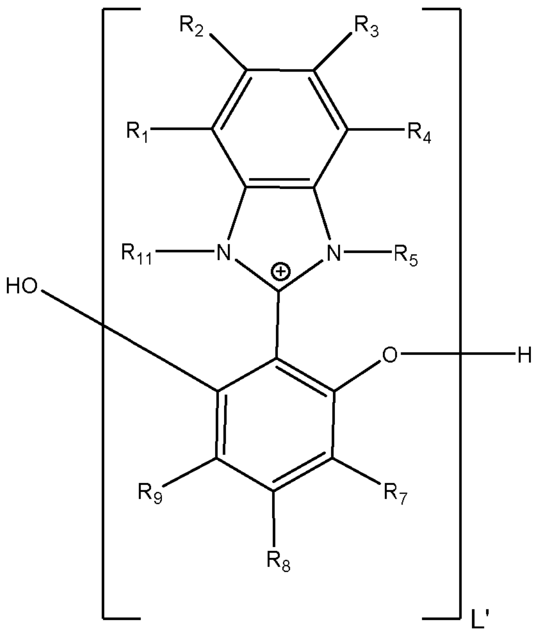

(IVb)

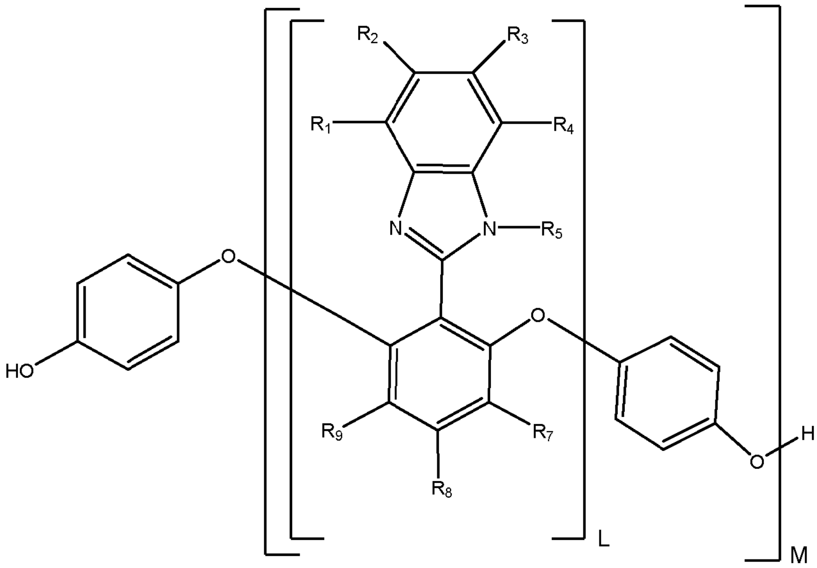

(IVc)

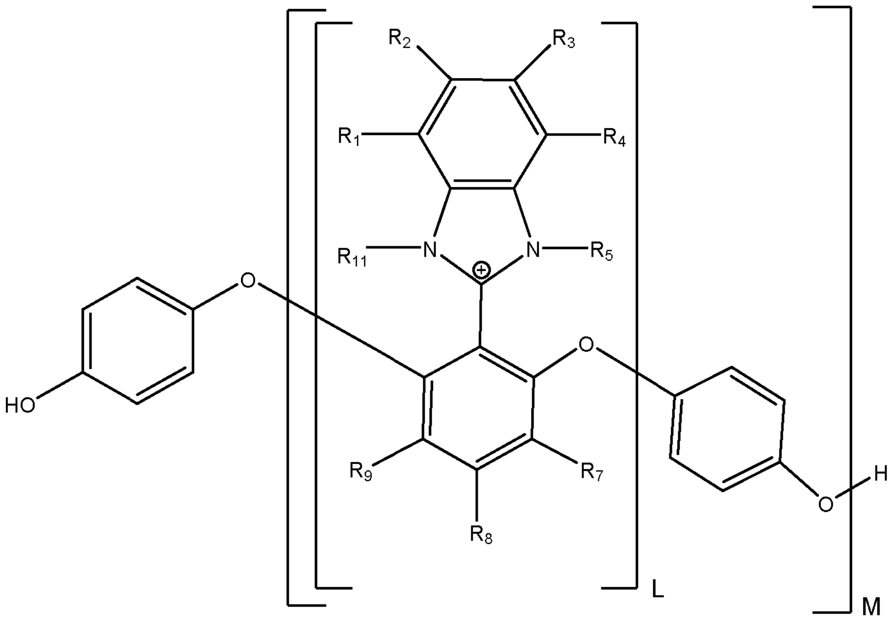

(IVd)

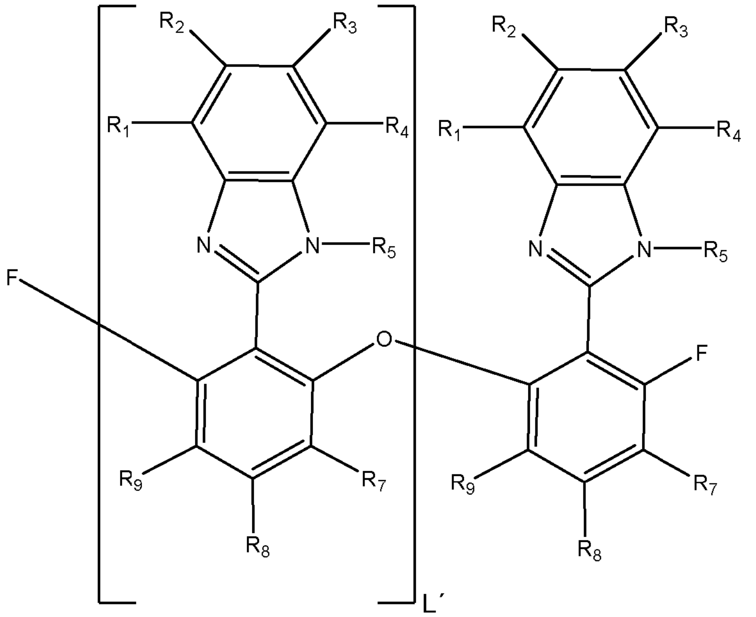

(IVe)

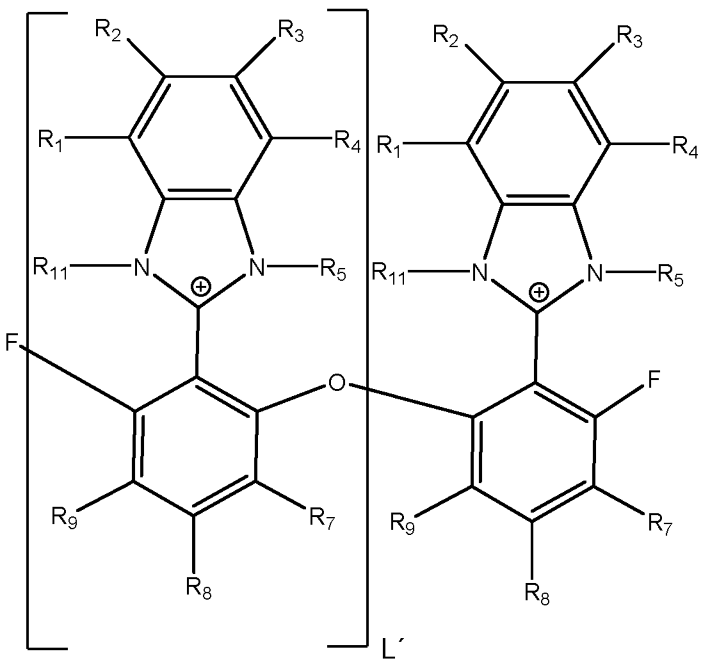

(IVf)

mit $R_1$, $R_2$, $R_3$, $R_4$, $R_5$, $R_7$, $R_8$ und $R_9$ wie vorstehend definiert, L = 1 bis 25, vorzugsweise 1 bis 15, bevorzugt 1 bis 9 und L' = 2 bis 25, vorzugsweise 2 bis 15, bevorzugt 3 bis 9 und M = 1 bis 500.

Besonders bevorzugte erfindungsgemäße Verbindungen sind Verbindungen der Formel (Va) oder (Vb)

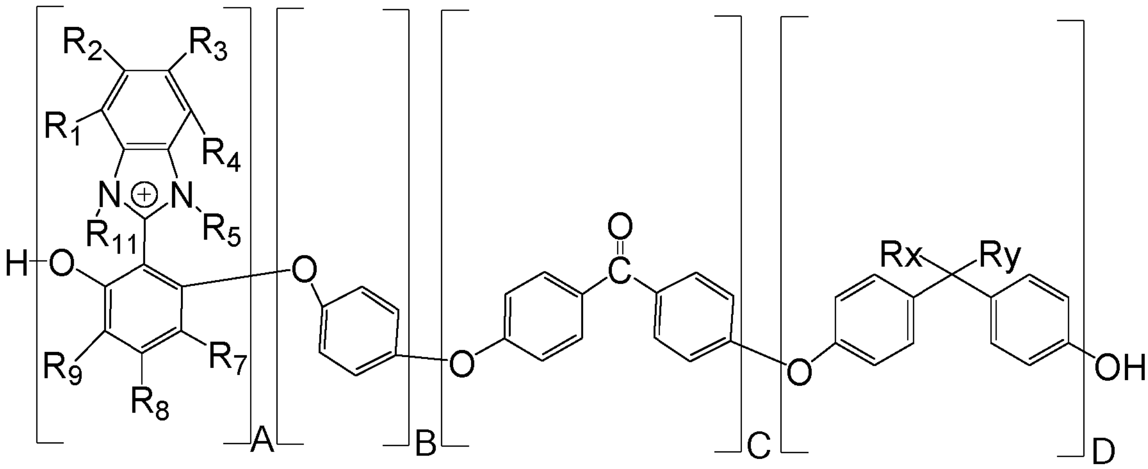

(Va)

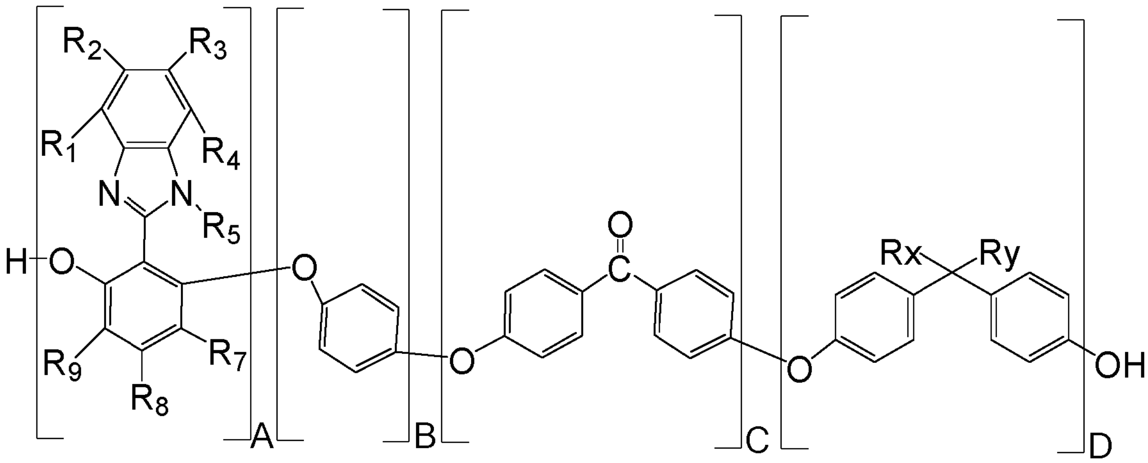

(Vb)

mit $R_1$, $R_2$, $R_3$, $R_4$, $R_5$, $R_7$, $R_8$, $R_9$ und $R_{11}$ wie vorstehend definiert und Rx und Ry gleich oder verschieden $-CH_3$ oder $-CF_3$ und A = 5 bis 500, B = 5 bis 500, C = 1 bis 500 und D = 0 bis 1000, wobei die mit den Indizes A, B, C und D gekennzeichneten Einheiten blockweise oder statistisch verteilt in der Verbindung auftreten können.

Ganz besonders bevorzugte Verbindungen sind Verbindungen der Formeln (VIa) bis (VId)

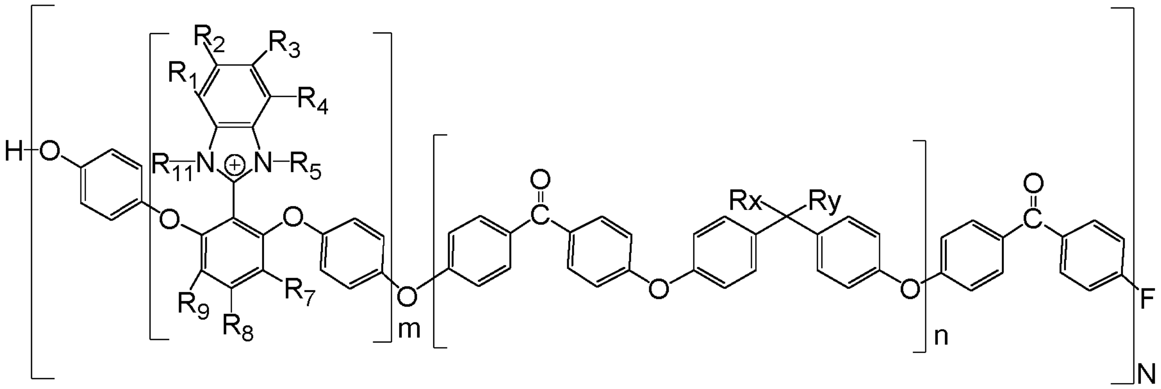

(VIa)

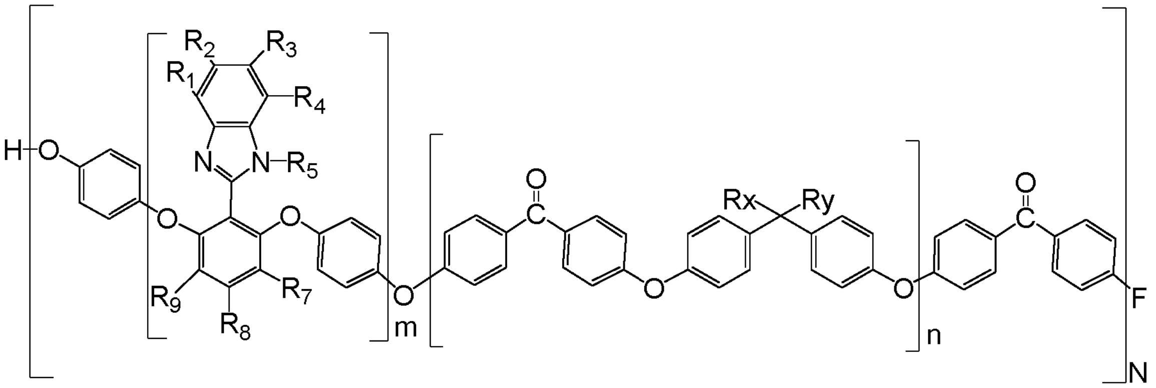

(VIb)

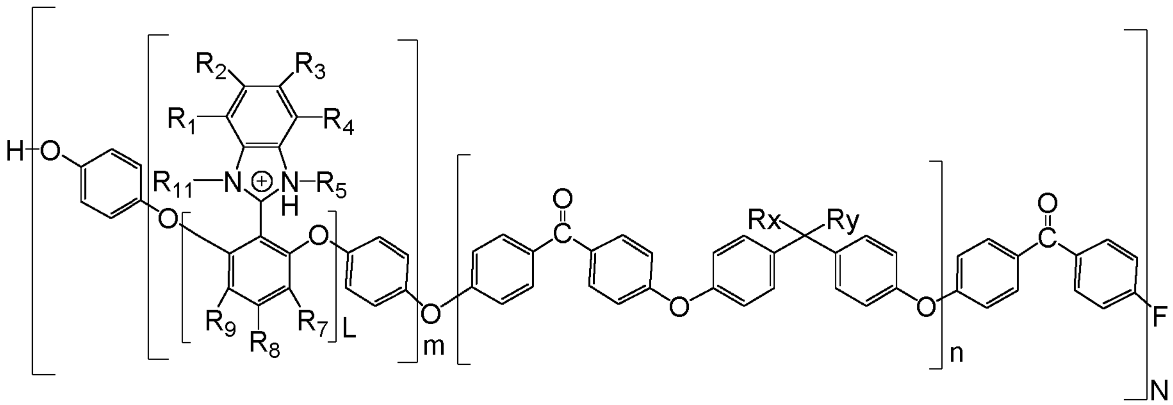

(VIc)

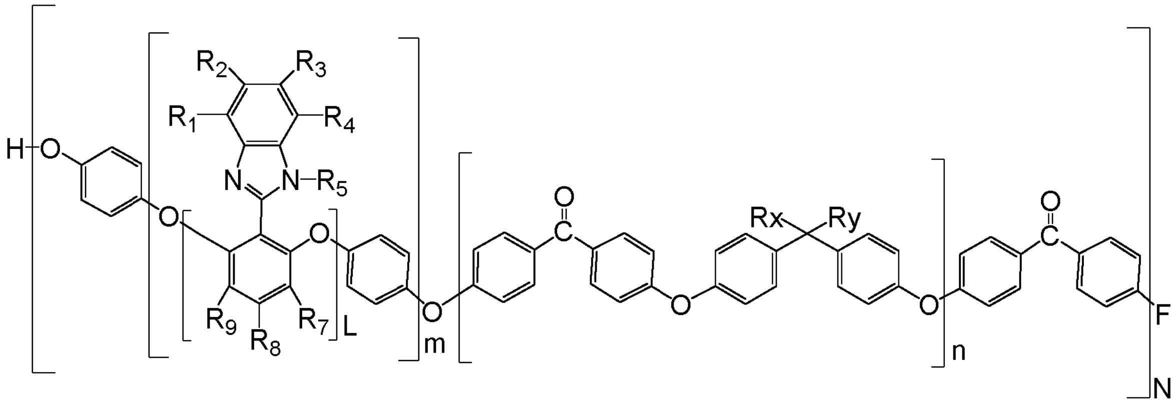

(VId)

mit $R_1$, $R_2$, $R_3$, $R_4$, $R_5$, $R_7$, $R_8$, $R_9$ und $R_{11}$ wie vorstehend definiert, L = 1 bis 25, vorzugsweise 3 bis 15, bevorzugt 3 bis 9, und Rx und Ry gleich oder verschieden $-CH_3$ oder $-CF_3$ und m = 1 bis 500, n = 1 bis 500, vorzugsweise 5 bis 50 und N = 1 bis 500, wobei die mit den Indizes m, n und N gekennzeichneten Einheiten blockweise oder statistisch verteilt in der Verbindung auftreten können.

Die erfindungsgemäßen Verbindungen können zum Beispiel mit dem nachfolgend beschriebenen Verfahren erhalten werden.

Das erfindungsgemäße Verfahren zur Herstellung von den voranstehend beschriebenen erfindungsgemäßen Verbindungen zeichnet sich dadurch aus, dass es einen Schritt (1) enthält, in welchem eine Verbindung der Formel (X)

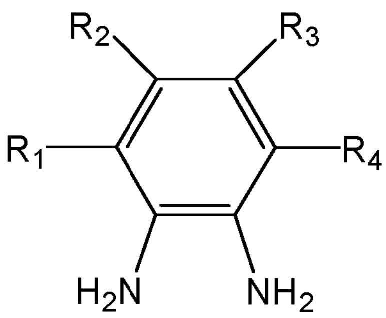

(X)

mit $R_1$ bis $R_4$ wie vorstehend definiert, mit einer Verbindung der Formel (XI)

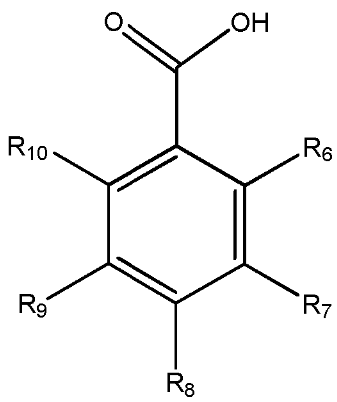

(XI)

mit $R_6$ bis $R_{10}$ wie vorstehend definiert, mit der Maßgabe, dass $R_6$ und $R_{10}$ = -F, zu einer Verbindung (XII)

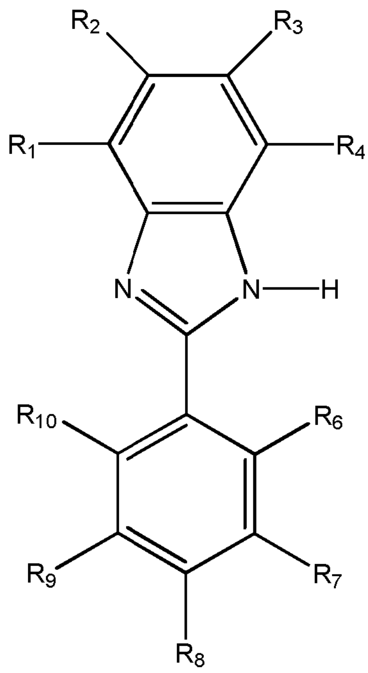

(XII)

umgesetzt wird,

und die Verbindung der Formel (XII) mit einem Methylierungsreagenz oder Trifluormethylierungsreagenz umgesetzt wird, unter Erhalt einer Verbindung der Formel (Ia), wie oben definiert, wobei $R_5$ = Alkyl- oder Perfluoralkyl-, vorzugsweise Methyl-, Ethyl-, Propyl-, iso-Propyl, n-Butyl-, tert-Butyl- oder Trifluormethylrest, bevorzugt gleich Methylrest und $R_6$ und $R_{10}$ = -F ist.

Vorzugsweise wird Schritt (1) so durchgeführt, dass Polyphosphorsäure und Methansulfonsäure in einem temperierbaren Reaktionsgefäß bei einer Temperatur von 40 bis 80 °C, vorzugsweise von 55 bis 65 °C vorgelegt wird und zu dieser Masse die Verbindung der Formel (X) unter Rühren und vorzugsweise unter Erwärmen des Reaktionsgemisches auf90 bis 130 °C, bevorzugt 105 bis 115 °C, zugegeben wird. Nachdem diese Masse vorzugsweise homogenisiert ist, erfolgt die Zugabe der Verbindung der Formel (XI), vorzugsweise ebenfalls unter Rühren. Zu dieser Reaktionsmasse wird vorzugsweise eine organisches Lösungsmittel, bevorzugt o-Xylol gegeben und die Temperatur des Reaktionsgemisches, vorzugsweise innerhalb von 3 Stunden, auf 140 bis 170 °C, bevorzugt auf 150 bis 160 °C erhöht und anschließend für vorzugsweise 30 bis 60 Stunden, bevorzugt 35 bis 45 Stunden bei dieser Temperatur belassen. Anschließend erfolgt eine Abkühlung, vorzugsweise auf 65 bis 95 °C, bevorzugt 75 bis 85 °C. Bei Erreichen dieser Temperatur wird vorzugsweise Wasser zugegeben. Die Zugabe erfolgt vorzugsweise in der Weise, dass die Temperatur in dem genannten Temperaturbereich bleibt. Nach Zugabe der vollständigen Menge Wasser, die vorzugsweise 100 bis 500 Volumen-% des im Reaktor befindlichen Reaktionsgemisches beträgt, wird der Reaktorinhalt vorzugsweise auf 10 bis 30 °C, bevorzugt 15 bis 25 °C und besonders bevorzugt 20 °C abgekühlt. Nach vorzugsweise 1 bis 5 stündigem, bevorzugt 1,5 bis 2,5 stündigem Rühren wird das erhaltene Reaktionsgemisch vorzugsweise einem Trennverfahren unterzogen, bei dem Feststoffe von Flüssigkeiten getrennt werden. Die abgetrennten Feststoffe werden vorzugsweise mit Wasser verrührt und unter Kühlung, so dass die Temperatur vorzugsweise von 30 bis 60 °C, bevorzugt 40 bis 50 °C beträgt, wird eine Lauge, vorzugsweise NaOH zugegeben, bis das Reaktionsgemisch einen pH-Wert von vorzugsweise 10 erreicht hat. Nach weiterem Rühren wird der Feststoff aus dem Gemisch wieder abgetrennt, mit Wasser gewaschen und getrocknet. Es wird eine Verbindung der Formel (XII) erhalten.

Zum Erhalten einer Verbindung der Formel (Ia) mit Re und $R_{10}$ = -F und $R_5$ gleich oder verschieden Alkyl- oder Perfluoralkyl-, vorzugsweise Methyl-, Ethyl-, Propyl-, iso-Propyl, n-Butyl-, tert-Butyl- oder Trifluormethylrest, vorzugsweise Methyl- oder Trifluormethylrest, bevorzugt Methylrest, kann die Verbindung der Formel (XII) z. B. in einem Reaktionsgefäß mit einem Alkylierungsreagenz, bevorzugt Dimethylcarbonat, $K_2CO_3$ und DMAc in Kontakt gebracht werden und anschließend durch Erwärmen auf eine Temperatur von 90 bis 130 °C, vorzugsweise unter Rühren, zur Reaktion gebracht werden. Vorzugsweise wird die Reaktion über einen Zeitraum von 10 bis 25 Stunden, bevorzugt von 15 bis 20 Stunden durchgeführt. Es kann vorteilhaft sein, zum Beenden der Reaktion Wasser dem Reaktionsgemisch zuzugeben und nach der Zugabe von Wasser vorzugsweise für 0,5 bis 2 Stunden weiter zu rühren. Es wird eine Verbindung der Formel (Ia) mit $R_5$ = -Methyl erhalten.

Das erfindungsgemäße Verfahren weist vorzugsweise einen Verfahrensschritt (2) auf, bei dem eine Verbindung

der Formel (Ia), wie oben definiert, mit Re und $R_{10}$ = -F und $R_5$ = Alkyl- oder Perfluoralkyl-, vorzugsweise Methyl-, Ethyl-, Propyl-, iso-Propyl, n-Butyl-, tert-Butyl- oder Trifluormethylrest ist, mit einem Silan, vorzugsweise $(CH_3)_3SiO^-K^+$ und anschließender Hydrolyse mit KOH oder NaOH unter Erhalt einer Verbindung der Formel (Ia) mit $R_{10}$ oder $R_6$ =-OH, nachfolgend als Verbindung der Formel (Ia‘) bezeichnet, umgesetzt wird.

Es kann vorteilhaft sein, wenn das erfindungsgemäße Verfahren einen Verfahrensschritt (3) aufweist, bei dem eine Verbindung der Formel (Ia) mit $R_1$ bis $R_5$ und $R_7$ bis $R_9$ wie oben definiert, und $R_6$ und $R_{10}$ = Fluor, mit mindestens einer Verbindung der Formel (Ia‘) mit $R_1$ bis $R_5$ und $R_7$ bis $R_9$ wie für Formel (Ia) definiert, zu einem Oligomeren der Formel (Ia") mit $R_1$ bis $R_5$ und $R_7$ bis $R_9$ wie für Formel (Ia) definiert und L’ = 2 bis 25, vorzugsweise 2 bis 15, bevorzugt 2 bis 9 umgesetzt wird.

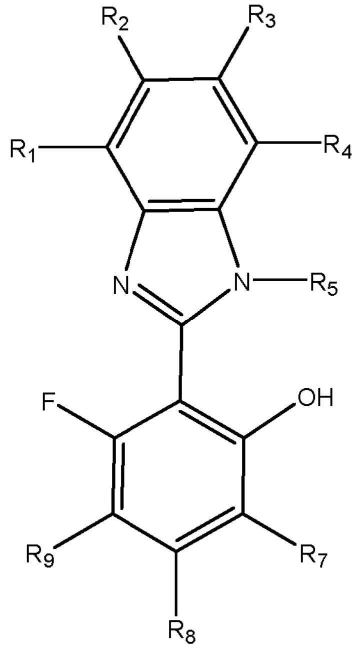

(Ia‘)

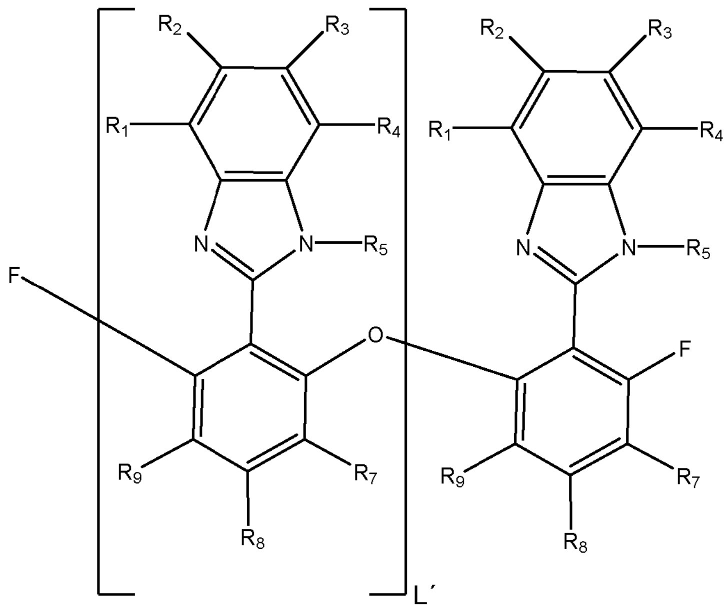

(Ia“)

Das erfindungsgemäße Verfahren weist bevorzugt einen Verfahrensschritt (4) auf, bei dem eine Verbindung der Formel (Ia) oder (Ia"), wie oben definiert, mit einem Diol, vorzugsweise Hydrochinon umgesetzt wird.

Die Umsetzung in Verfahrensschritt (4) erfolgt vorzugsweise wie nachfolgend beschrieben in einem Reaktionsgefäß, in welches die Verbindung der Formel (Ia) oder (Ia"), $K_2CO_3$, Hydrochinon mit DMAc gespült werden. Vorzugsweise unter Inertgasatmosphäre, bevorzugt Stickstoffatmosphäre wird das Gemisch vorzugsweise unter Rühren zum Sieden erhitzt. Am Kopf des Reaktionsgefäßes werden gegebenenfalls entstehendes Methanol und/oder Wasser abgenommen.

Das erfindungsgemäße Verfahren weist vorzugsweise einen Verfahrensschritt (5) auf, bei dem das Umsetzungsprodukt von Diol, vorzugsweise Hydrochinon, mit einer Verbindung der Formel (Ia) oder Formel (Ia"), wie oben definiert, mit einer Verbindung der Formel (XIII)

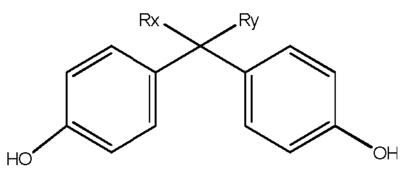

(XIII)

mit Rx und Ry gleich oder verschieden Alkyl- oder Perfluoralkyl-, vorzugsweise Methyl-, Ethyl-, Propyl-, iso-Propyl, n-Butyl-, tert-Butyl- oder Trifluormethylrest, bevorzugt $-CH_3$ oder $-CF_3$, und einer Difluoroverbindung, vorzugsweise 4,4’-Difluorbenzophenon oder einer Verbindung der Formel (XIV)

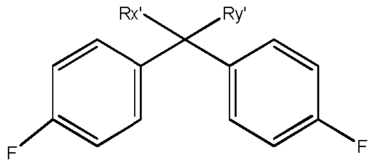

(XIV)

mit Rx' und Ry' gleich oder verschieden Alkyl- oder Perfluoralkyl-, vorzugsweise Methyl-, Ethyl-, Propyl-, iso-Propyl, n-Butyl-, tert-Butyl- oder Trifluormethylrest, bevorzugt-$CH_3$ oder -$CF_3$, umgesetzt wird.

Vorzugsweise wird der Verfahrensschritt (5) wie folgt durchgeführt: Bei Raumtemperatur werden 4,4'-Difluorbenzophenon (DFBP) und eine Verbindung der Formel (XIII), vorzugsweise Bisphenol A, gegebenenfalls unter Zugabe eines Lösungsmittels, bevorzugt DMAc und optional Zugabe von $K_2CO_3$, zu der in Verfahrensschritt (4) erhaltenen Reaktionsmasse gegeben. Dieses Reaktionsgemisch wird zum Sieden gebracht und von 10 bis 30 Stunden, vorzugsweise von 12 bis 25 Stunden am Sieden gehalten, wobei während der Reaktion, insbesondere zu Beginn der Reaktion entstehendes Reaktionswasser abgezogen wird.

Nach dem Erkalten kann das Polymer aus Wasser ausgefällt werden. Es kann vorteilhaft sein, das ausgefällte Polymer zu zerkleinern, z.B. unter Verwendung eines Ultraturrax, und anschließend ein oder mehrfach mit Wasser und optional anschließend mit Ethanol zu waschen (auszulaugen). Vorteilhafterweise wird das Polymer nach dem Waschen bei erhöhter Temperatur und Unterdruck, bevorzugt bei 125 bis 160 °C und einem leichten Vakuum (Unterdruck von kleiner 500 mbar, vorzugsweise von etwa 200 mbar) getrocknet.

Vorzugsweise wird das in dem Verfahrensschritt (5) erhaltene Produkt in einem weiteren Verfahrensschritt (6) mit einem Alkylierungsreagenz, bevorzugt Methylierungsreagenz umgesetzt. Vorzugsweise wird Verfahrensschritt (6) so durchgeführt, dass das in Verfahrensschritt (5) erhaltene Polymer in einem Lösungsmittel, z. B. N,N-Dimethylacetamid, vorzugsweise bei einer Temperatur von 30 bis 70 °C, bevorzugt 45 bis 55 °C , bevorzugt unter leichtem Rühren gelöst wird. Nach dem Abkühlen der erhaltenen Lösung wird diese auf eine Temperatur von 20 bis 40 °C, vorzugsweise 25 bis 35 °C temperiert und es wird unter Rühren Iodmethan, z.B. über eine Spritze, zugetropft. Nach einem Zeitraum von vorzugsweise 0,25 bis 5 Stunden, bevorzugt 1,5 bis 2,5 Stunden, in denen vorzugsweise weiter gerührt wird, wird mit einer Vakuumpumpe ein Unterdruck von kleiner 500 mbar, vorzugsweise von etwa 200 mbar angelegt, um überschüssiges Iodmethan abzuziehen.

Bevorzugte erfindungsgemäße Verfahren sind solche, die einen oder mehrere der vorzugsweise oder bevorzugt eingesetzten Verfahrensschritte, vorzugsweise alle Verfahrensschritte (1) bis (6), aufweisen.

Die oben beschriebenen erfindungsgemäßen polymeren Verbindungen können z. B. als Anionen leitende Membran, zur Herstellung einer Anionen leitenden Membran oder zur Herstellung eines Bauteils, welches in einem elektrochemischen Verfahren, vorzugsweise ausgewählt aus Elektrolyse, Elektrodialyse und Brennstoffzellentechnik eingesetzt wird.

Entsprechend zeichnen sich erfindungsgemäße Membranen und Elektrolyseure dadurch aus, dass sie ein erfindungsgemäße Verbindung, Oligomer oder Polymer, vorzugsweise Polymer, aufweisen.

Zur Herstellung einer (erfindungsgemäßen) Membran, insbesondere einer Anionen leitenden Membran, kann direkt die in Verfahrensschritt (6) erhaltene Lösung eingesetzt werden. Vorzugsweise erfolgt zunächst die Herstellung einer Membran in der Weise, dass die benötigte Menge an Polymerlösung mit einer Spritze oder ähnlichem aufgenommen und vorzugsweise durch einen PTFE-Filter (zur Abtrennung von evtl. nicht gelösten Polymerteilchen) auf eine vorgeheizte, vorzugsweise auf eine auf 30 °C bis 40 °C vorgeheizte Glasplatte aufgetragen wird. Für die Beschichtung der Glasplatte wird vorzugsweise ein Rakel mit einem Spalt von bevorzugt 350 $\mu$m benutzt, welcher automatisch mit einer Geschwindigkeit von 1 bis 50 mm/s bevorzugt 2 bis 5 mm/s über die Glasplatte gezogen wird. Die aufgetragene Nassschicht wird vorzugsweise unter einem Inertgas, bevorzugt Stickstoff für mindestens 10 Stunden, bevorzugt von 12 bis 10 Stunden vorgetrocknet. Das Vortrocknen kann bei Raumtemperatur oder erhöhter Temperatur erfolgen. Vorzugsweise erfolgt das Vortrocknen bei Raumtemperatur. Nach dem Vortrocknen erfolgt vorzugsweise eine Trocknung bei vermindertem Druck, vorzugsweise bei einem Druck von kleiner 200 $mbar_{abs}$, bevorzugt kleiner-gleich 100 $mbar_{abs}$ und bei einer Temperatur oberhalb von 25 °C, bevorzugt im Bereich von 40 bis 80 °C, bevorzugt 55 bis 65 °C.

Zur Herstellung der Anionen leitenden Membran kann die so hergestellte Membran z. B. mit 0,5 M KOH oder KCl Lösungen (wässrig) behandelt werden. Dazu kann die Membran mehrfach, vorzugsweise 3 Mal jeweils für 1 Stunde bei 60 °C in die entsprechende Lösung eingelegt und anschließend in frischer Lösung über Nacht bei Raumtemperatur gelagert werden. Es kann vorteilhaft sein, wenn die so behandelten Membrane anschließend mit deionisiertem Wasser abgespült und mehrfach, vorzugsweise 3 Mal jeweils für 1 Stunde bei 60 °C in frischen Portionen des deionisierten Wassers eingelegt werden und anschließend in einer frischen Portion deionisiertem Wasser bei Raumtemperatur gelagert werden.

In den nachfolgend aufgeführten Beispielen wird die vorliegende Erfindung beispielhaft beschrieben, ohne

dass die Erfindung, deren Anwendungsbreite sich aus der gesamten Beschreibung und den Ansprüchen ergibt, auf die in den Beispielen genannten Ausführungsformen beschränkt sein soll.

**Beispiele:**

**Beispiel 1: Synthese von 2-(2',6'-Difluorophenyl)-Benzimidazol (DFP-BI)**

In ein 3 L Reaktionsgefäß mittemperaturgeregeltem Ölbad, mechanischem Rührwerk, einem Einlass für Stickstoff und einem langen, aufsteigenden Glasrohr als Kühler wurden 1600 g Polyphosphorsäure überführt und das Ölbad auf 120 °C erwärmt. Anschließend wurden 632 g von 2,6-Difluorobenzoesäure (DFBA) langsam eingerührt. In diese Mischung wurden im Verlauf einer Stunde 432 g ortho-Phenylendiamin eingerührt. Die Temperatur des Ölbades wurde einen Tag auf 125 °C, einen Tag auf 135 °C, 3 Tage auf 145 °C und 1 Tag auf 150 °C gehalten. Die an den kälteren Oberflächen des Reaktors in langen Nadeln sublimierte DFBA wurde mehrmals durch Zugabe einiger MililiterTetrahydrofuran (THF), welches verdampfte und an den kälteren Stellen kondensierte, in die Reaktionsmasse zurück gespült. Die Reaktionsmasse wurde abgekühlt und bei etwa 80 °C mit Wasser langsam auf ein Gesamtvolumen von 3 L verdünnt. Dabei fiel das Dihydrogenphosphat des DFP-BI aus der Lösung aus. Dieser Niederschlag wurde abgenutscht, zwei Mal mit jeweils zwei Litern Wasser aufgeschlämmt und abgenutscht. Der noch nasse Filterkuchen wurde in einer Lösung von 250 g NaOH in 1,5 L Wasser aufgeschlämmt und dabei das DFP-BI aus seinem Dihydrogenphosphat frei gesetzt. Nach einer Stunde Rühren wurde abgenutscht. Der Filterkuchen wurde in 2 L Wasser geschlämmt und mit Ammoniumhydroxydlösung auf einen pH-Wert von 9 gebracht. Diese Reinigung wurde mit reinem Wasser wiederholt. Der abgenutschte Filterkuchen wurde bei 120 °C bei 30 mbar über Nacht getrocknet. Rohausbeute: 810 g leicht rosa Produkt, das sind 88 % der theoretischen Ausbeute.

Dieses Produkt kann durch Umkristallisieren aus THF gereinigt werden. Das solcherart gereinigte Produkt wurde mittels $^1H$- und $^{13}C$-NMR untersucht und eindeutig als 2-(2',6'-Difluorophenyl)-Benzimidazol identifiziert.

**Beispiel 2: Methylierung von DFP-BI zu MeDFP-BI**

Die Apparatur bestand aus einem 1 L Vierhalskolben mit mechanischem Rührwerk, Stickstoffbeaufschlagung, Aluminiumheizblock mit Temperaturregelung und einer Füllkörperkolonne mit 35 cm Länge und 2 cm Durchmesser, die mit Raschig-Ringen gefüllt war. Am Kopf der Kolonne war ein Kühler mit einstellbarem Rücklaufverhältnis und Kondensatentnahme aufgesetzt.

Bei Raumtemperatur wurden 34,50 g von DFP-BI aus Beispiel 1 (umkristallisiert aus THF), 26,1 g von Dimethylcarbonat in 75 g Dimethylacetamid (DMAc) gelöst, 10,35 g vom gemahlenen $K_2CO_3$ zugegeben und 1 Stunde bei 95 °C Heizblocktemperatur, 15 Stunden bei 100 °C und noch 1 Stunde bei 125 °C zur Reaktion gebracht. Anschließend wurde auf 100 °C Blocktemperatur geregelt, 9,0 ml Wasser zugegeben und noch 1 Stunde gerührt, um überschüssiges Dimethylcarbonat zu zersetzen.

**Beispiel 3: Oligomerisierung von MeDFP-BI**

Der Ansatz aus Beispiel 2 wurde auf Raumtemperatur abgekühlt und 13,8 g von $K_2CO_3$ (gemahlen und bei 400 °C getrocknet) sowie 17,60 g von Hydrochinon mit 50 g DMAc in die Apparatur gespült, 20 Minuten mit Stickstoff geflutet und danach unter Rühren zum Sieden erhitzt, indem der Heizblock auf 225 °C geregelt wurde. Der Reihe nach wurden am Kolonnenkopf zuerst Methanol und dann Wasser abgezogen. Bei der Reaktion entstandenes Wasser wurde laufend über die Kolonne entfernt. Nach 18 Stunden wurde eine Aufschlämmung von 10,3 g von $K_2CO_3$ in 20 g DMAc zugegeben und weitere 5 Stunden bei Siedehitze zur Reaktion gebracht.

**Beispiel 4: Polymerisation**

Ansatz aus Beispiel 3 wurde auf Raumtemperatur abgekühlt und es wurden 32,70 g von 4,4'-Difluorobenzophenone (DFBP), 31,92 g von Bisphenol A (BPA;) sowie 10,3 g von $K_2CO_3$ und 130 g DMAc zugegeben und weitere 20 Stunden am Sieden gehalten, wobei speziell zu Beginn das Reaktionswasser am Kolonnenkopf abgezogen wurde. Dann wurden noch 3,45 g von $K_2CO_3$ zur bereits viskosen Lösung zugegeben und weitere 3 Stunden auf Siedetemperatur gehalten.

**Beispiel 5: Aufarbeitung des Polymers**

Nach dem Erkalten der Reaktionsmasse aus Beispiel 4 wurde die Reaktionsmasse in 2 L Wasser gefällt, das ausgefallene Polymer mit Hilfe eines Ultraturrax zerkleinert und 2 mal mit Wasser bei 80 °C ausgelaugt. Zum Schluss

wurde das Polymer noch mit 500 ml Ethanol bei 60 °C ausgelaugt. Das abgenutschte Polymer wurde bei 150 °C im Vakuum getrocknet. Ausbeute: 106 g fast weißes Produkt.

**Beispiel 6:**

**Synthese von 1-Methyl, 2-(2',6'-Difluoro-phenyl)-Benzimidazol (MeDFP-BI)**

In ein 3 L Reaktionsgefäß mit von der Innentemperatur geregeltem Ölbad, mechanischem Rührwerk, einem Einlass für Stickstoff und einem Rücklaufkühler wurden 1500 g Dimethylformamid ( DMF ), 100 g von $K_2CO_3$ und 4,5 mol Dimethylcarbonat (DMC) eingebracht.

690 g von DFP-BI (Rohprodukt aus Beispiel 1) wurden in zwei Tranchen zu 400 g und 290 g aufgeteilt. Die erste Tranche wurde in den Reaktor eingebracht. Die Reaktionslösung wurde zuerst eine halbe Stunde auf 95 °C und weitere 5 Stunden auf 100 °C geregelt. Nach dem Abkühlen wurde die zweite Tranche zugegeben, eine halbe Stunde auf 95 °C, weitere 5 Stunden auf 100 °C und drei Stunden auf 125 °C geregelt.

Nach dem Abkühlen auf Raumtemperatur wurde die Reaktionslösung in 5 L Wasser verrührt. Dabei schied sich eine feste Masse ab. Diese wurde abfiltriert, mit Wasser gewaschen und bei 90 °C im Vakuum über Nacht getrocknet.

Rohausbeute: 660 g

**Beispiel 7:**

**Synthese von 1-Methyl, 2-(2'-Hydroxy-6'-Fluoro-phenyl)-Benzimidazol (MeHyFP-BI)**

Die Apparatur war ein 3 L Reaktionsgefäß mit mechanischem Rührwerk, Stickstoffbeaufschlagung, Ölbad mit Temperaturregelung und einer Füllkörperkolonne mit 35 cm Länge und 2 cm Durchmesser, die mit Raschig-Ringen gefüllt war. Am Kopf der Kolonne war ein Kühler mit einstellbarem Rücklaufverhältnis und Kondensatentnahme aufgesetzt.

Der Reaktor wurde bei Raumtemperatur mit 488 g von MeDFP-BI aus Beispiel 6, 300 g KOH in Form von Flakes, 100 g Hexamethyldisiloxan (HMDS) und 800 g Sulfolan beschickt. Das Ölbad wurde auf 105 °C geregelt. Bald darauf begann die Lösung aufgrund der exothermen Reaktion stark zu sieden. Bald darauf verringerte sich die verdampfende Menge von HMDS, weshalb die Temperatur des Ölbades langsam erhöht wurde, sodass die Lösung immer siedete.

Nach 7 Stunden wurde etwas von HMDS am Kolonnenkopf abgezogen und danach abgekühlt. Bei Unterschreiten von etwa 70 °C wurde die Reaktionslösung mit 1,5 L Wasser verdünnt. Die Lösung war leicht trüb und wurde bei Raumtemperatur über eine G3-Fritte filtriert. Das klare Filtrat wurde mit Essigsäure neutralisiert. Dabei fiel das MeHyFP-BI aus, das abgenutscht und mit Wasser gewaschen wurde. Der Filterkuchen wurde in 2,5 L Wasser aufgeschlämmt und mit konzentrierter Salzsäure bis pH-Wert von ca. 0 angesäuert. Beim Aufheizen auf 80 °C löste sich das MeHyFP-BI als Hydrochlorid auf und die Lösung wurde heiß filtriert. Aus dem erkaltenden Filtrat schied sich das Hydrochlorid in Form weißer Kristalle ab. Es wurde abgenutscht. Das Filtrat wurde mit Ammoniaklösung neutralisiert, wobei MeHyFP-BI ausfiel. (Fraktion 2).

Das abgenutschte Hydrochlorid wurde in Wasser geschlämmt, MeHyFP-BI ebenfalls mit Ammoniaklösung frei gesetzt und abfiltriert (Fraktion 1). Beide Fraktionen wurden bei 100 °C im Vakuum über Nacht getrocknet.

| | |
|---|---|
| Fraktion 1: | 431 g; gemäß HPLC sehr sauber |
| Fraktion 2: | 40,5 g; leicht rosa, ist wahrscheinlich unrein |

**Beispiel 8:**

**Trimerisierung von MeDFP-BI und MeHyFP-BI aus Beispiel 6 zum Makromer**

In einen 500 ml Dreihalskolben mit mechanischem Rührwerk, einer Füllkörperkolonne ( L = 35 cm, D=2 cm) mit Kolonnenkopf, Aluminiumheizblock mit Temperaturregelung und Stickstoffspülung wurden 0,0500 mol ( 12,20 g) MeDFP-BI, 0,1000 mol ( 24,20 g) MeHyFP-BI, 0,1014 mol ( 14.0 g) $K_2CO_3$ mit 70 g DMAc überführt und 30 Minuten mit Stickstoff gespült. Anschließend wurde der Heizblock auf 225 °C erhitzt und die Lösung begann zu sieden. Gebildetes Reaktionswasser wurde laufend am Kolonnenkopf abgezogen. Nach 20 Stunden wurde der Heizblock auf Raumtemperatur abgekühlt.

**Beispiel 9: Oligomerisierung vom Makromer aus Beispiel 7 mit Hydroquinon zum Oligomer**

Zu der erkalteten Reaktionsmischung aus Beispiel 8 wurden unter Stickstoff 6,60 g von Hydrochinon sowie 8,40 g von $K_2CO_3$ und 25 g DMAc zugegeben und der Heizblock wieder auf 225 °C erhitzt. Gebildetes Reaktionswasser wurde laufend am Kolonnenkopf abgezogen. Nach 16 Stunden wurde die Reaktionslösung auf Raumtemperatur abgekühlt.

**Beispiel 10: Polymerisation vom Oligomer aus Beispiel 9 zum Block-Copolymer**

Zu der erkalteten Reaktionsmischung aus Beispiel 9 wurden unter Stickstoff 10,90 g von 4,4'-Difluorobenzophenon sowie von einer Gesamtmenge von 13,44 g von 2,2-(4'-Hydroxyphenyl)-Hexafluoropropan (BPA-6F) die ersten 12,00 g sowie 8,40 g von $K_2CO_3$ und 70 g DMAc zugegeben. Der Heizblock wurde wieder auf 225 °C erhitzt. Gebildetes Reaktionswasser wurde laufend am Kolonnenkopf abgezogen. Nach 3 Stunden Reaktionszeit wurden 1,00 g von BPA-6F sowie 1,00 g von $K_2CO_3$ und 10 g DMAc zugegeben und die Temperatur des Heizblockes auf 200 °C geregelt. Nach weiteren 3 Stunden wurden die restlichen 0.44 g von BPA-6F zugegeben, der Heizblock auf 190 °C geregelt und noch 3 weitere Stunden auf dieser Temperatur gehalten. Danach wurde auf Raumtemperatur abgekühlt und das Polymers analog Beispiel 5 aufgearbeitet.

**Beispiel 11: Quarternisierung des Polymers mit Jodmethan**

Zur Quarternisierung des Polymers wurden 15 g des Produktes aus Beispiel 10 in 45 g N,N-Dimethylacetamid im Kolben unter leichtem Rühren bei 50 °C gelöst und ca. eine Stunde bis zum vollständigen Lösen des Polymers gerührt. Nach dem Abkühlen der Lösung auf 30 °C wurden 6,6 g Iodmethan durch langsames Zutropfen über eine Spritze zugegeben und die Lösung wurde für weitere 2 Stunden gerührt. Das nicht verbrauchte Iodmethan wurde anschließend mit einer Vakuumpumpe bei 200 mbar abgezogen und die Gasphase wurde durch zwei seriell angeordnete, mit 30 Massen-%ige, wässrige KOH Lösung gefüllte Gaswaschflaschen zur Zerstörung des Iodmethans geleitet.

**Beispiel 12: Quarternisierung des Polymers mit Dimethylsulfat**

ZurQuartemisierung des Polymers wurden 3,0 g des Produktes aus Beispiel 10 in 7 g DMF im Kolben unter Rühren bei 50 °C gelöst. Nach dem Abkühlen der Lösung auf 25 °C wurden 1,1 g von Dimethylsulfat zugetropft und verrührt. Die exotherme Reaktion begann zuerst langsam, erwärmte das Reaktionsgemisch auf über 45 °C und war bei dieser Temperatur nach wenigen Minuten komplettiert.

**Beispiel 13: Herstellung der Membran**

Die im Beispiel 11 beschriebene Lösung des quaternisierten Polymers wurde direkt zur Herstellung der Membran verwendet. Die benötigte Menge an Polymerlösung wurde mit einer Spritze aufgenommen und direkt durch einen PTFE-Filter (zur Abtrennung von evtl. nicht gelösten Polymerteilchen) auf eine auf 30 °C vorgeheizte Glasplatte aufgetragen. Für die Beschichtung der Glasplatte wurde ein Rakel mit einem Spalt von 350 $\mu$m benutzt, welcher automatisch mit einer Geschwindigkeit von 5 mm/s über die Glasplatte gezogen wurde. Die aufgetragene Nassschicht wurde für 16 Stunden unter Stickstoff bei Raumtemperatur vorgetrocknet und anschließend für 6 Stunden bei 60 °C unter Vakuum getrocknet.

**Beispiel 14: Ionentausch der Membran**

Die im Beispiel 13 hergestellte Membran wurde ionengetauscht, d.h. die durch die Quatemisierung des Polymers vorliegenden Iodidionen wurden mit Chlorid- bzw. Hydroxidionen ausgetauscht. Dafür wurden die zugeschnittenen Membranproben in wässrige 0,5 M KOH Lösung 3 Mal jeweils für 1 Stunde bei 60 °C eingelegt und anschließend in frischer 0,5 M KOH Lösung über Nacht bei Raumtemperatur gelagert. Nach dem Ionentausch wurden die Membranproben mit deionisiertem Wasser abgespült und 3 Mal jeweils für 1 Stunde bei 60 °C in frischen Portionen des deionisierten Wassers eingelegt. Anschließend wurden die Membranproben in einer frischen Portion des deionisierten Wassers über Nacht bei Raumtemperatur gelagert.

**Beispiel 15: Bestimmung der ionischen Leitfähigkeit der Membran**

Die in-plane, i.e. flächige, ionische Leitfähigkeit der ionengetauschten Membranproben wurde mittels Impedanzspektroskopie (EIS) in üblicher 4-Elektrodenanordnung gemessen. Die Membranprobe wurde in einer kommerziellen BT-112 Zelle (Bekk Tech LLC) so befestigt, dass die beiden äußeren Pt Drähte unter der Probe und die beiden Mittleren über der Probe platziert waren. Die BT-112 Zelle wurde zwischen 2 PTFE Platten befestigt und mit DI Wasser gefüllt. Die Temperatur des DI Wassers wurde mittels eines Wasserbads kontrolliert und DE Wasser wurde permanent durch die Zelle gepumpt. Die Berechnung des Wiederstandes ($R_{Membran}$) wurde durch das Fitten des EIS-Spektrums mittels weit verbreitetem R(RC) Randles Equivalentschaltkreises durchgeführt. Die ionische Leitfähigkeit ($\sigma$) der Membranprobe ergibt sich aus der Gleichung (1):

$$\sigma = L/(R_{Membran}*A)\ (1)$$

mit L der Abstand zwischen Pt Drähten (5 mm) und A der Fläche der Membranprobe zwischen den zwei äußeren Pt-Drähten.

**Beispiel 16: Bestimmung der Wasseraufnahme der Membran**

Die ionengetauschten Membranproben (jeweils 3 Proben pro getestete Membran) wurden für 24 Stunden in einem Vakuumofen bei 40 °C und 25 mbar getrocknet, anschließend im Exsikkator auf Raumtemperatur abgekühlt und gewogen. Für die Messung der Wasser-aufnahme wurden die Membranproben für 24 Stunden in auf 25 °C temperiertes, deionisiertes Wasser ausgelagert. Anschließend wurde das Gewicht jeder Probe neu bestimmt. Hierfür wurde noch anhaftendes Wasser mit Hilfe eines Filterpapiers von der Membran entfernt. Jede Messung wurde 3 Mal wiederholt und ein Mittelwert ± Standardabweichung wurden berechnet. Die Wasseraufnahme (WA) ergibt sich aus Gleichung (2):

$$WA = (m_{nass} - m_{trocken})/m_{trocken} * 100\%\quad (2)$$

mit $m_{nass}$ der Masse der Probe nach dem Quellen und $m_{trocken}$ der Trockenmasse der Probe.

**Beispiel 17: Bestimmung des Quellverhaltens der Membran**

Die ionengetauschten Membranproben (jeweils 3 Proben pro getestete Membran) wurden für 24 Stunden in einem Vakuumofen bei 40 °C und 25 mbar getrocknet, anschließend im Exsikkator auf Raumtemperatur abgekühlt und solche Parameter wie die Probenlänge, die Probenbreite sowie die Probendicke wurden bestimmt. Für die Bestimmung des Quellverhaltens wurden die Membranproben für 24 Stunden in auf 25 °C temperiertem, deionisiertem Wasser gelagert. Anschließend wurden die Probenlänge, die Probenbreite sowie die Probendicke neu bestimmt. Hierfür wurde noch anhaftendes Wasser mit Hilfe eines Filterpapiers von der Membran entfernt. Jede Messung wurde 3 Mal wiederholt und ein Mittelwert ± Standard-abweichung wurden berechnet. Das Quellverhalten (bezeichnet als dimensionale Stabilität, DS) in Länge, Breite und Dicke ergibt sich aus Gleichung (3):

$$DS = (x_{nass} - x_{trocken})/x_{trocken} * 100\%\quad (3)$$

mit $x_{nass}$ der Länge, Breite oder Dicke der Probe nach dem Quellen und $x_{trocken}$ der Trockenlänge, Trockenbreite oder Trockendicke der Probe.

**Beispiel 18: Bestimmung der mechanischen Belastbarkeit der Membran**

Die ionengetauschten Membranproben (jeweils 3 Proben pro getestete Membran) wurden für 24 h in deionisiertem Wasser gelagert. Vor dem Einbau der Probe in das Messsystem (DMA 8000 mit Wasserbad) wurden Breite und Dicke jeder Membranprobe mehrfach bestimmt. Die Durchführung der Messung sieht wie folgt aus - Membranprobe wird zwischen zwei sich senkrecht gegenüberstehenden Klammem mit einer statischen Vorspannung eingebaut. Um eine statische Vorspannung auf die Probe zu bringen, wird während des Einbaus, der Abstand zwischen den Klammem (auch als freie Weglänge l bezeichnet) um ca. 1 mm verringert. Die Probe wird zwischen beiden Klammem fixiert und anschließend die ursprüngliche freie Weglänge wiederhergestellt, wodurch die Probe gedehnt wird. Der gesamte Messaufbau wird in einem beheizbaren Wasserbad mit deionisiertem Wasser versenkt, sodass die Probe vollständig von Wasser umgeben wird. Die Messprozedur umfasst die Untersuchung der Probe in einem Temperaturbereich zwischen Raumtemperatur (ca. 23 °C) und 80 °C bei einer angelegten Heizrate von 2 K/min. Innerhalb dieses Temperaturintervalls wird die Probe sinusförmig mit einer Dehnung $\varepsilon$ von 0,1 % bei einer Frequenz von 1 Hz kontinuierlich belastet. Die Dehnung in % ergibt sich aus Gleichung (4):

$$\varepsilon = \Delta l/l \quad (4)$$

mit $\Delta l$ der Probendehnung in mm und l der freien Weglänge. Bei einer freien Weglänge von l = 10 mm ergibt sich für $\varepsilon$ = 0,1 % eine Dehnung von 0,01 mm. Über einen Kraftsensor wird die für die vorgegebene Dehnung erforderliche Spannung detektiert. Die Ergebnisse der Ausprüfung sind in Tabelle 1 angegeben.

**Tabelle 1:** Ergebnisse der Ausprüfungen

| | Wasseraufnahme [%] | Dimensionale Stabilität[1] [%] | Ionische Leitfähigkeit[2] [mS/cm] | Ionische Leitfähig keit[3] [mS/cm] | Mechanische Belastbarkeit[4] [GPa] |
|---|---|---|---|---|---|
| Membran 1 | 8,1 ± 1,1 | 4,6 ± 1,1 | 6,4 ± 0,4 | - | 1,429 ± 0,101 |
| Membran 2 | 3,2 ± 0,9 | 4,2 ± 0,1 | 51,3 ± 1,2 | - | 0,853 ± 0,037 |
| FAA-3 | 28,4 ± 8,8 | 13,1 ± 2,7 | 30,9 ± 3,4 | - | 0,086 ± 0,01 |
| Nation N-115 | 14,0 ± 0,7 | 5,1 ± 0,5 | - | 110,7 ± 4,8 | 0,085 ± 0,01 |

**Erläuterungen**

- Membran 1 wurde aus dem im Beispiel 5 beschriebenen und anschließend quaternisierten Polymer hergestellt
- Membran 2 wurde aus dem im Beispiel 10 beschriebenen und anschließend quaternisierten Polymer hergestellt
- FAA-3 ist eine kommerziell erhältliche anionenleitfähige Membran der Firma FUMATECH BWT GmbH
- Nafion N-115 ist eine kommerziell erhältliche kationenleitfähige Membran der Firma The Chemours Company (USA)
- [1]Diese Daten beziehen sich auf die Änderung der Dicke der Membran
- [2]Diese Daten beziehen sich auf die Leitfähigkeit der Membran in OH-Form, gemessen @ 60 °C
- [3]Diese Daten beziehen sich auf die Leitfähigkeit der Membran in H+-Form, gemessen @ 60 °C
- [4]Alle Membrane außer Nation (H+-Form) wurden in OH-Form @ 60 °C gemessen

**Patentansprüche**

**1.** Verbindung enthaltend mindestens einen Baustein der Formel (Ia) oder (Ib)

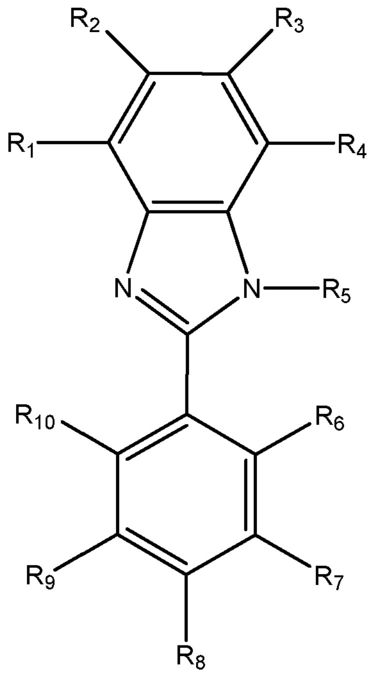

(Ia)

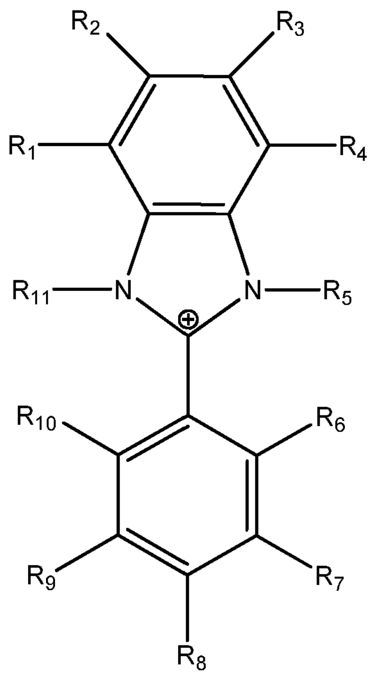

(Ib)

wobei $R_1$, $R_2$, $R_3$, und $R_4$ gleich oder verschieden-H, oder eine beliebige Gruppe, vorzugsweise gleich -H, $R_5$ und $R_{11}$ gleich oder verschieden Alkyl- oder Perfluoralkylrest,
$R_6$, und $R_{10}$ gleich oder verschieden Oligomer- oder Polymerrest und
$R_7$, $R_8$ und $R_9$ gleich oder verschieden -H oder eine beliebige Gruppe, vorzugsweise gleich -H sind, **dadurch gekennzeichnet, dass** mindestens einer der Reste $R_6$ oder $R_{10}$ ein Oligomer- oder Polymerrest ist, der über ein Sauerstoffatom an das Ringkohlenstoffatom angebunden ist.

**2.** Verbindung nach Anspruch 1, **dadurch gekennzeichnet, dass** die Verbindung ein Oligomer oder Polymer ist, welches mindestens 2 Bausteine der Formel (Ia) oder (Ib), vorzugsweise (Ib) aufweist.

**3.** Verbindung nach Anspruch 1 oder 2, **dadurch gekennzeichnet, dass** $R_6$ und $R_{10}$ einen Polymerrest darstellen.

**4.** Verbindung nach einem der Ansprüche 1 bis 3, **dadurch gekennzeichnet, dass** die Verbindung eine Polymerverbindung ist, deren Polymerreste $R_6$ und $R_{10}$ einen oder mehrere der Bausteine der Formel (IIa) und/oder (IIb) und/oder (IIIa) und/oder (IIIb)

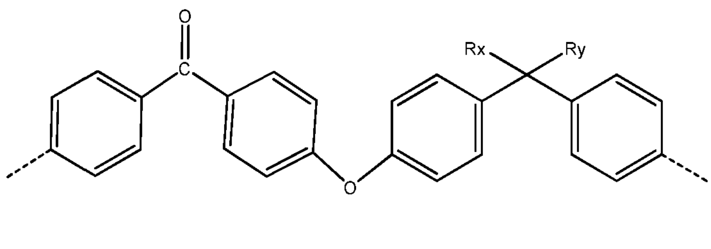

(IIa)

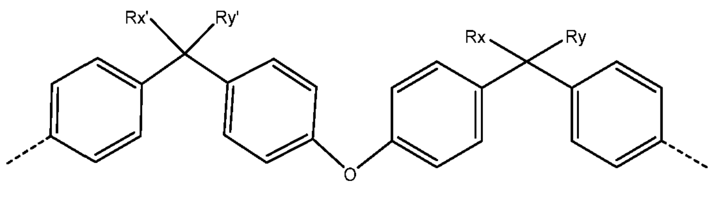

(IIb)

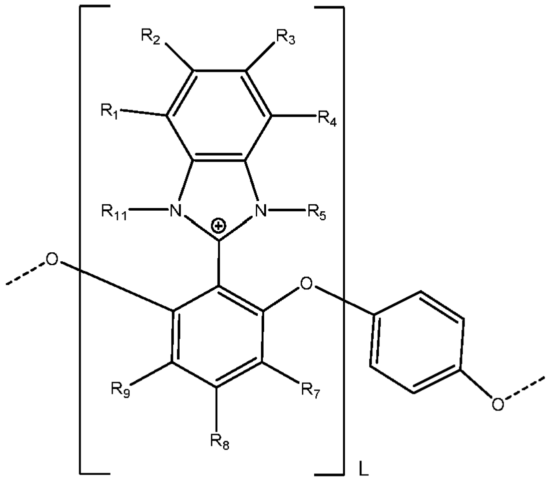

(IIIa)

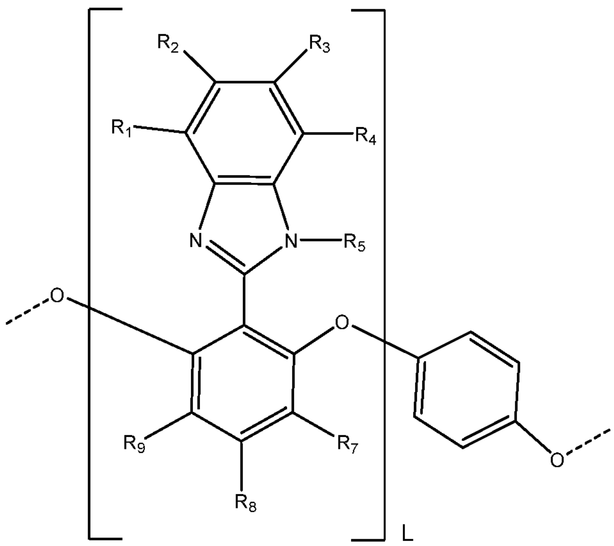

(IIIb)

aufweist, mit $R_1$, $R_2$, $R_3$, $R_4$, $R_5$, $R_7$, $R_8$, $R_9$ und $R_{11}$ wie in den vorangegangenen Ansprüchen definiert, L = 1 bis 25, vorzugsweise 1 bis 15, bevorzugt 1 bis 9, und Rx und Ry und Fbc‘ und Ry’ gleich oder verschieden Alkyl-, Phenyl- oder Perfluoralkylrest.

5. Verbindung nach einem der vorhergehenden Ansprüche, **dadurch gekennzeichnet, dass** die Verbindung eine Verbindung der Formel (IVa), (IVb), (IVc), (IVd), (IVe) oder (IVf)

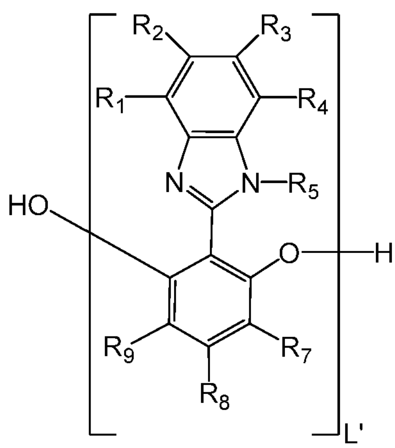

(IVa)

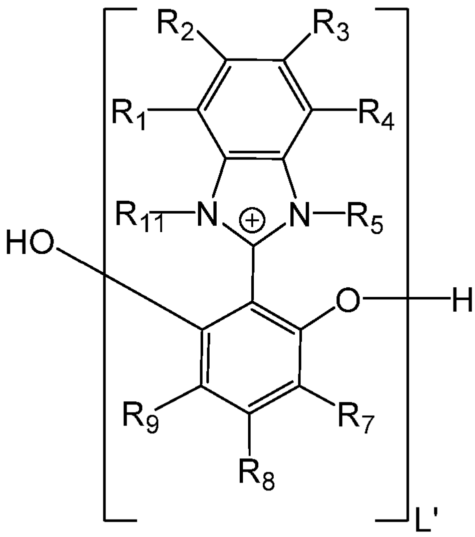

(IVb)

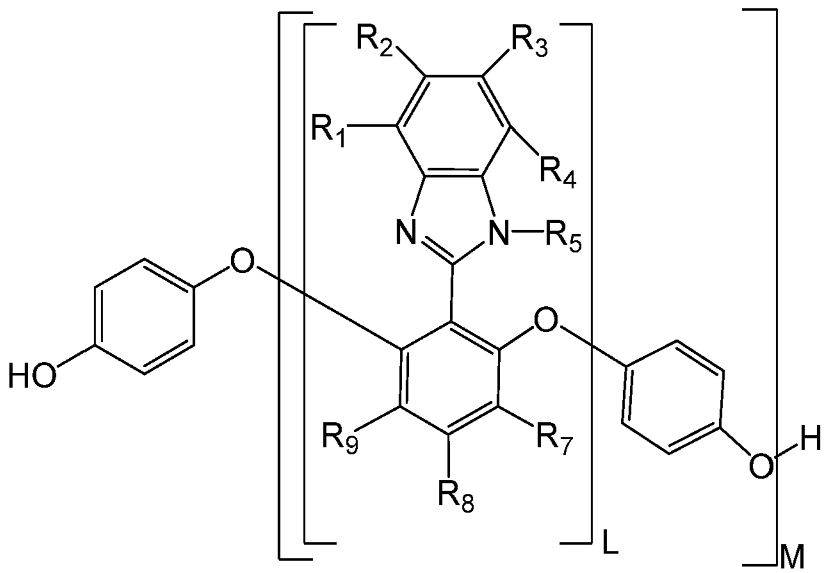

(IVc)

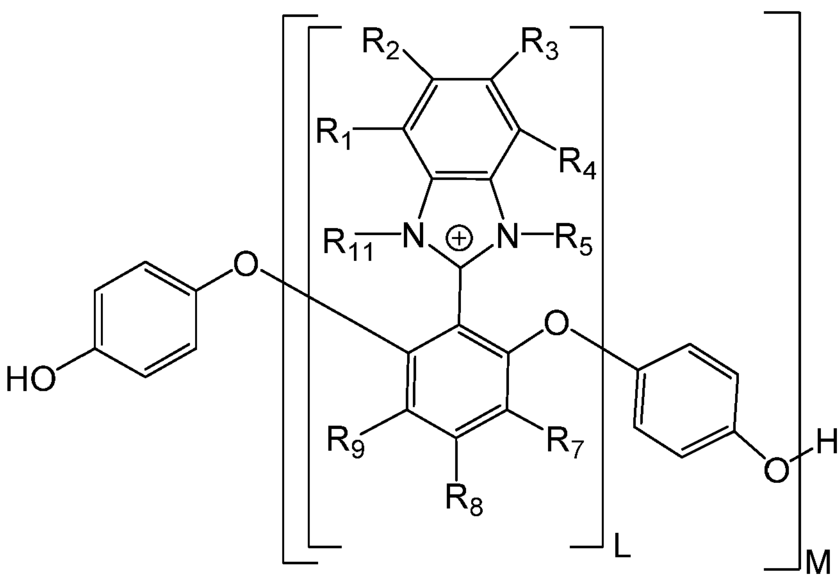

(IVd)

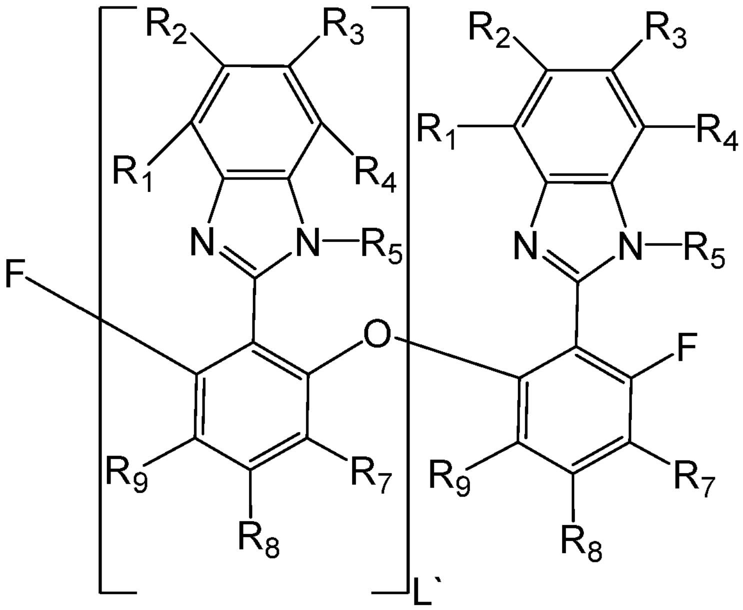

(IVe)

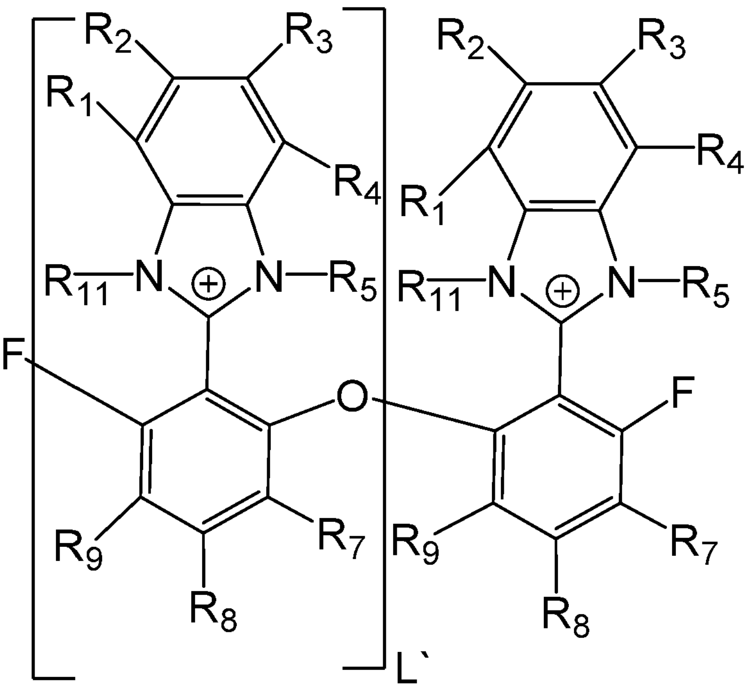

(IVf)

ist, mit $R_1$, $R_2$, $R_3$, $R_4$, $R_5$, $R_7$, $R_8$ und $R_9$ wie in den vorangegangenen Ansprüchen definiert, L = 1 bis 25, vorzugsweise 1 bis 15, bevorzugt 1 bis 9 und L' = 2 bis 25, vorzugsweise 2 bis 15, bevorzugt 3 bis 9 und M = 1 bis 500.

6. Verbindung nach einem der vorhergehenden Ansprüche, **dadurch gekennzeichnet, dass** die Verbindung eine Verbindung der Formel (Va) oder (Vb)

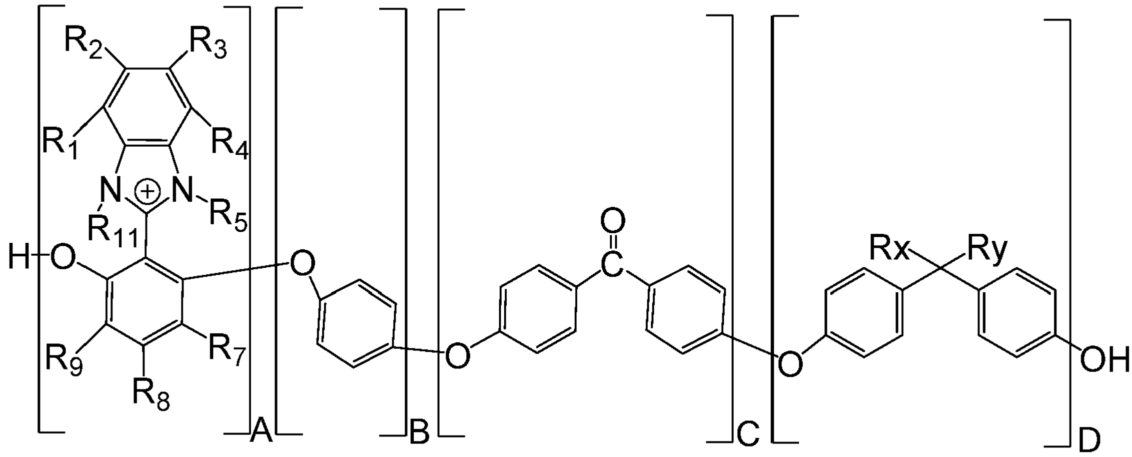

(Va)

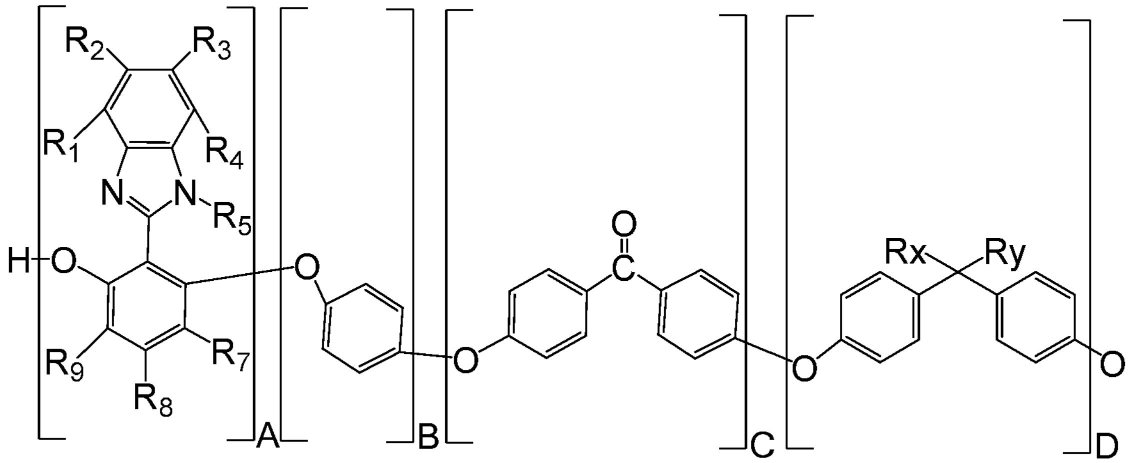

(Vb)

ist mit $R_1$, $R_2$, $R_3$, $R_4$, $R_5$, $R_7$, $R_8$, $R_9$ und $R_{11}$ wie in den vorangegangenen Ansprüchen definiert und Rx und Ry gleich oder verschieden $-CH_3$ oder $-CF_3$ und A = 5 bis 500, B = 5 bis 500, C = 1 bis 500 und D = 0 bis 1000, wobei die mit den Indizes A, B, C und D gekennzeichneten Einheiten blockweise oder statistisch verteilt in der Verbindung auftreten können.

**7.** Verbindung nach einem der vorgehenden Ansprüche, **dadurch gekennzeichnet, dass** die Verbindung eine Verbindung der Formeln (VIa) bis (VId)

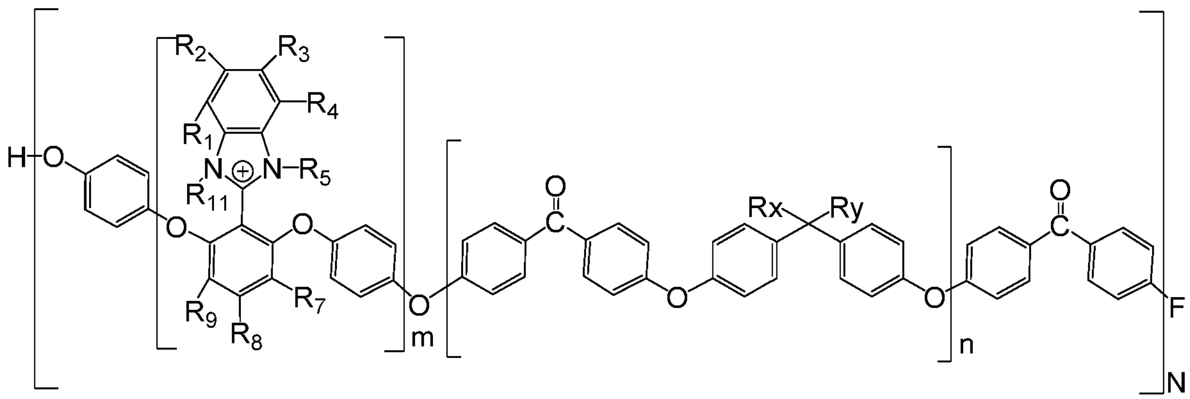

(VIa)

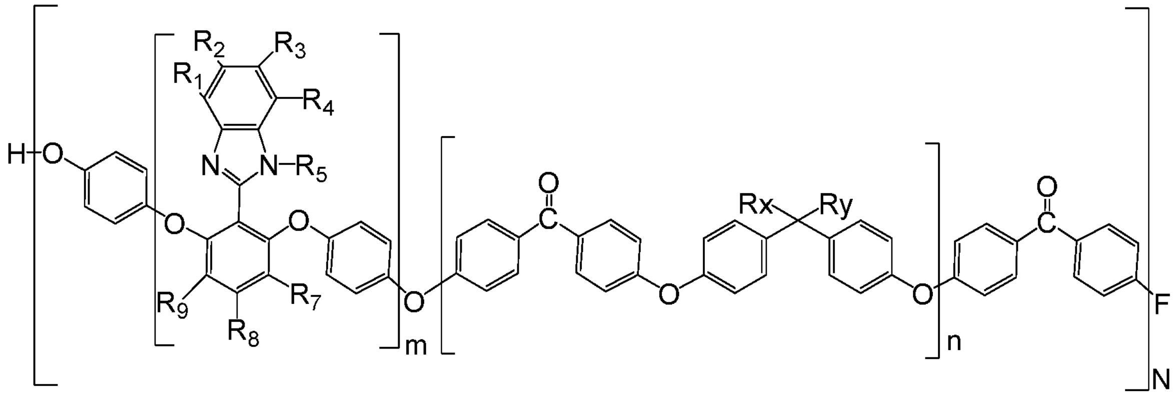

(VIb)

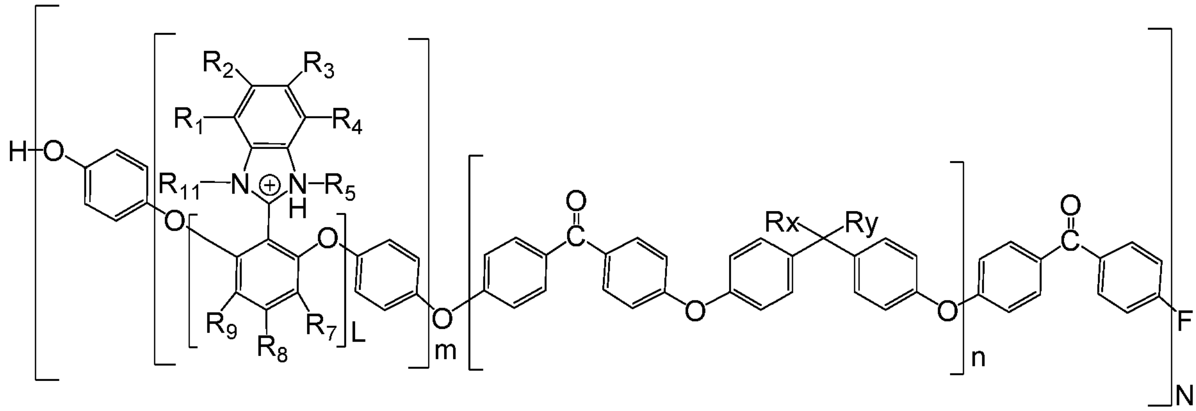

(VIc)

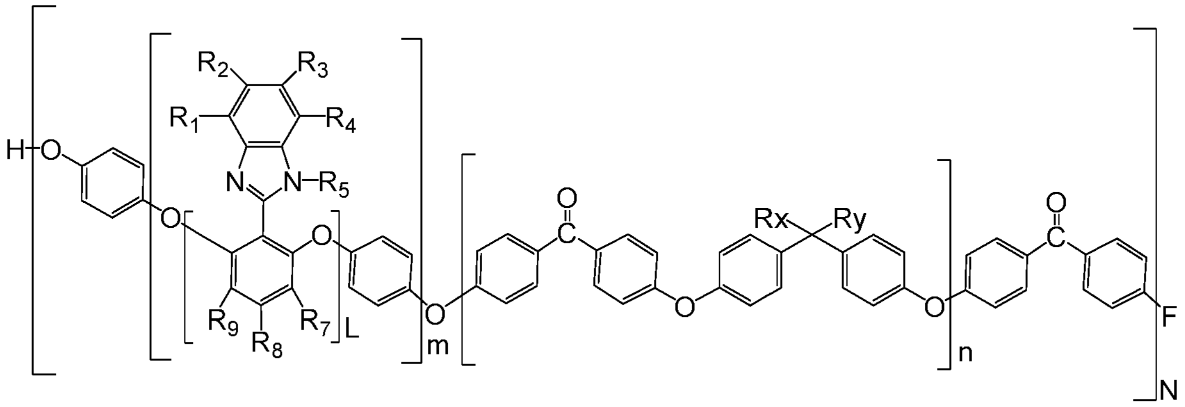

(VId)

ist mit $R_1$, $R_2$, $R_3$, $R_4$, $R_5$, $R_7$, $R_8$, $R_9$ und $R_{11}$ wie vorstehend definiert, L = 1 bis 25, vorzugsweise 3 bis 15, bevorzugt 3 bis 9, und Rx und Ry gleich oder verschieden $-CH_3$ oder $-CF_3$ und m = 1 bis 500, n = 1 bis 500, vorzugsweise 5 bis 50 und N = 1 bis 500, wobei die mit den Indizes m, n und N gekennzeichneten Einheiten blockweise oder statistisch verteilt in der Verbindung auftreten können.

**8.** Verfahren zur Herstellung von Verbindungen nach einem der Ansprüche 1 bis 7, **dadurch gekennzeichnet, dass** es einen Schritt enthält, in dem eine Verbindung der Formel (X)

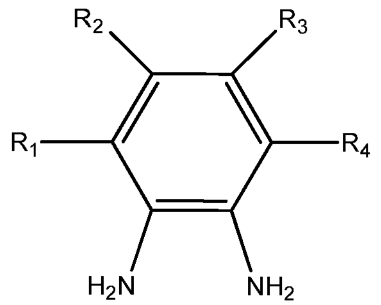

(X)

mit $R_1$ bis $R_4$ wie in einem der vorangegangenen Ansprüche definiert, mit einer Verbindung der Formel (XI)

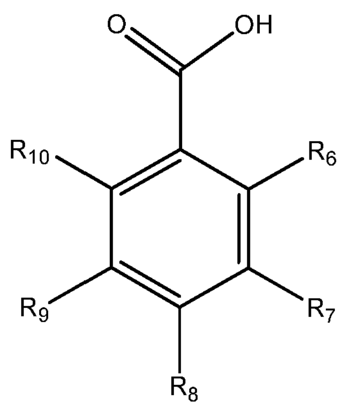

(XI)

mit $R_6$ bis $R_{10}$ wie in einem der vorangegangenen Ansprüche definiert, wobei $R_6$ und $R_{10}$ = -F, zu einer Verbindung (XII)

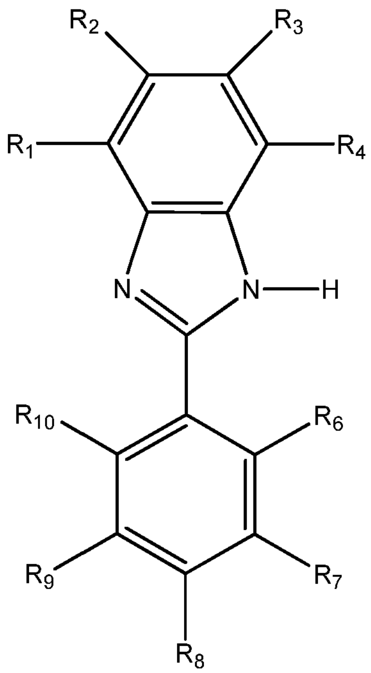

(XII)

umgesetzt wird,
und die Verbindung der Formel (XII) mit einem Methylierungsreagenz oder Trifluormethylierungsreagenz umgesetzt wird, unter Erhalt einer Verbindung der Formel (Ia), wie in Anspruch 1 definiert, wobei $R_5$ = Alkyl- oder Perfluoralkylrest und $R_6$ und $R_{10}$ = -F ist.

**9.** Verfahren nach Anspruch 8, **dadurch gekennzeichnet, dass** es einen Verfahrensschritt aufweist, bei dem eine Verbindung der Formel (Ia), wie in Anspruch 1 definiert, mit einem Silan unter Erhalt einer Verbindung der Formel (Ia) mit $R_{10}$ oder $R_6$ = -OH, nachfolgend als Verbindung der Formel (Ia‘) bezeichnet, umgesetzt wird.

**10.** Verfahren nach Anspruch 9, **dadurch gekennzeichnet, dass** es einen Verfahrensschritt aufweist, bei dem eine Verbindung der Formel (Ia) mit mindestens einer Verbindung der Formel (Ia‘) zu einem Oligomeren der Formel (Ia") umgesetzt wird.

**11.** Verfahren nach einem der Ansprüche 8 bis 10, **dadurch gekennzeichnet, dass** es einen Verfahrensschritt aufweist, bei dem eine Verbindung der Formel (Ia), wie in Anspruch 1 definiert, oder Formel (Ia"), wie in Anspruch 10 definiert, mit einem Diol umgesetzt wird.

**12.** Verfahren nach einem der Ansprüche 8 bis 11, **dadurch gekennzeichnet, dass** es einen Verfahrensschritt aufweist, bei dem das Umsetzungsprodukt einer Verbindung der Formel (Ia), wie in Anspruch 1 definiert, oder Formel (Ia"), wie in Anspruch 10 definiert, mit einem Diol, mit einer Verbindung der Formel (XIII)

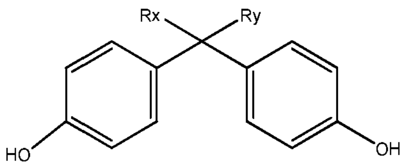

(XIII)

mit Rx und Ry gleich oder verschieden Alkyl- oder Perfluoralkylrest, und eine Difluorverbindung oder einer Verbindung der Formel (XIV)

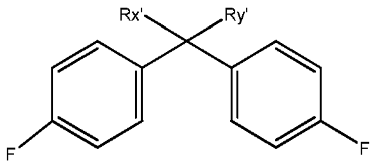

(XIV)

mit Fbc‘ und Ry’ gleich oder verschieden Alkyl- oder Perfluoralkylrest, umgesetzt wird.

**13.** Verfahren nach Anspruch 12, **dadurch gekennzeichnet, dass** das in dem Verfahrensschritt gemäß Anspruch 10 erhaltene Produkt mit einem Alkylierungsreagenz, vorzugsweise Methylierungsreagenz umgesetzt wird.

**14.** Verwendung eines Polymeren nach einem der Ansprüche 1 bis 7 zur Herstellung einer Anionen leitenden Membran.

**15.** Verwendung eines Polymeren nach einem der Ansprüche 1 bis 7 als Anionen leitende Membran oder zur Herstellung eines Bauteils, welches in einem elektrochemischen Verfahren, vorzugsweise ausgewählt aus Elektrolyse, Elektrodialyse und Brennstoffzellentechnik eingesetzt wird.

**16.** Elektrolyseur, **dadurch gekennzeichnet, dass** er ein Polymer gemäß einem der Ansprüche 1 bis 7 aufweist.

**17.** Anionen leitende Membran, enthaltend ein Polymer nach einem der Ansprüche 1 bis 7.

## Claims

**1.** Compound containing at least one unit of the formula (Ia) or (Ib)

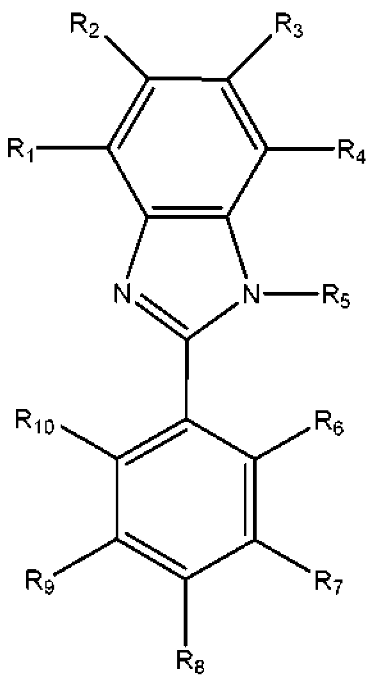

(Ia)

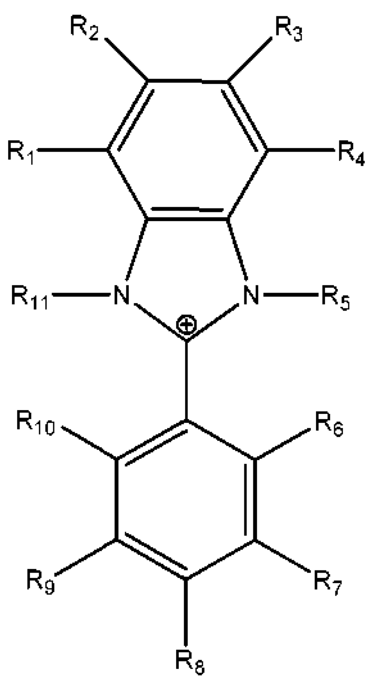

(Ib)

where $R_1$, $R_2$, $R_3$, and $R_4$ are the same or different and are -H or any desired group, preferably -H,
$R_5$ and $R_{11}$ are the same or different and are an alkyl or perfluoroalkyl radical,
$R_6$ and $R_{10}$ are the same or different and are an oligomer or polymer radical and
$R_7$, $R_8$und $R_9$ are the same or different and are -H or any desired group, preferably -H, **characterized in that** at least one of the $R_6$ or $R_{10}$ radicals is an oligomer or polymer radical attached via an oxygen atom to the ring carbon atom.

2. Compound according to Claim 1, **characterized in that** the compound is an oligomer or polymer having at least 2 units of the formula (Ia) or (Ib), preferably (Ib).

3. Compound according to Claim 1 or 2, **characterized in that** $R_6$ and $R_{10}$ represent a polymer radical.

4. Compound according to any of Claims 1 to 3, **characterized in that** the compound is a polymer compound wherein the polymer radicals $R_6$ and $R_{10}$ have one or more units of the formula (IIa) and/or (IIb) and/or (IIIa) and/or (IIIb)

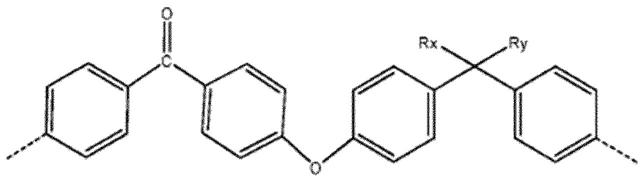

(IIa)

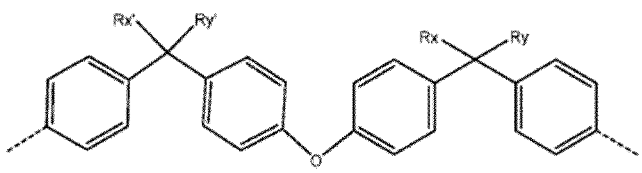

(IIb)

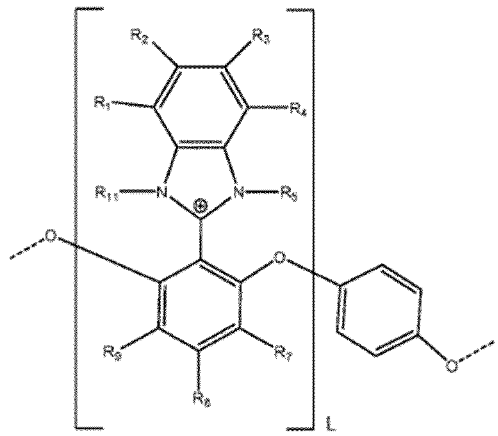

(IIIa)

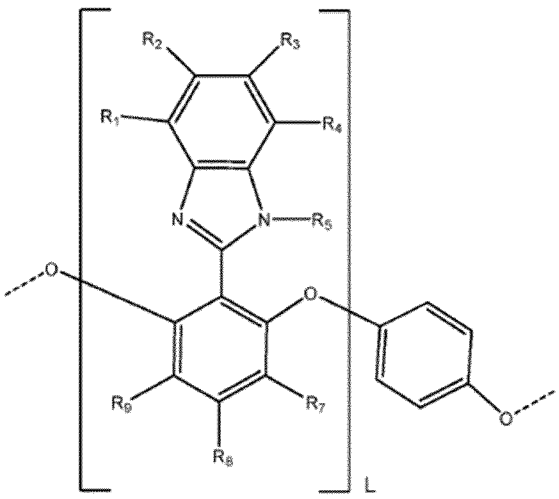

(IIIb)

with $R_1$, $R_2$, $R_3$, $R_4$, $R_5$, $R_7$, $R_8$, $R_9$ and $R_{11}$ as defined in the preceding claims, L = 1 to 25, preferably 1 to 15, more preferably 1 to 9, and where Rx and Ry and Rx' and Ry' are the same or different and are an alkyl, phenyl or perfluoroalkyl radical.

**5.** Compound according to any of the preceding claims, **characterized in that** the compound is a compound of the formula (IVa), (IVb), (IVc), (IVd), (IVe) or (IVf)

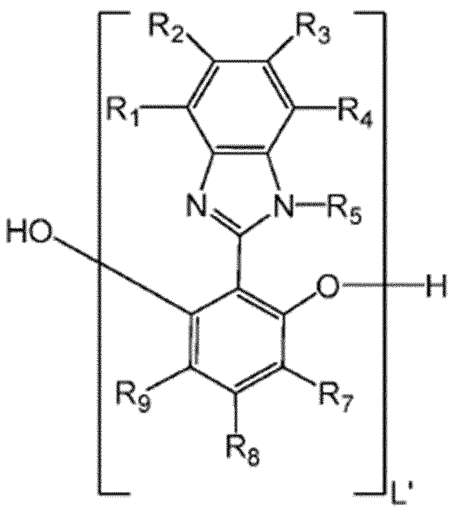

(IVa)

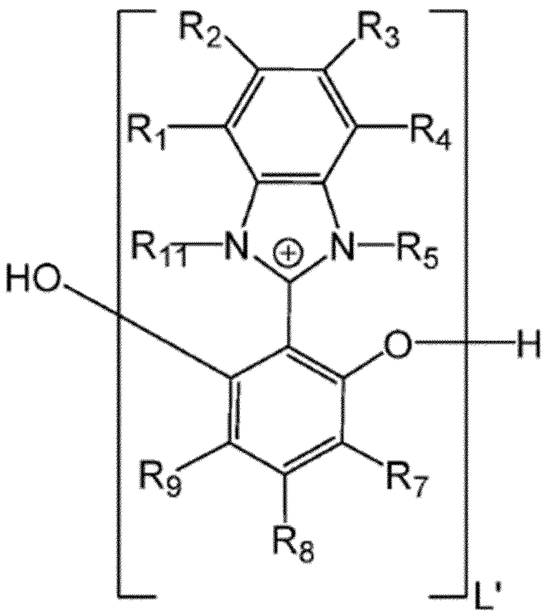

(IVb)

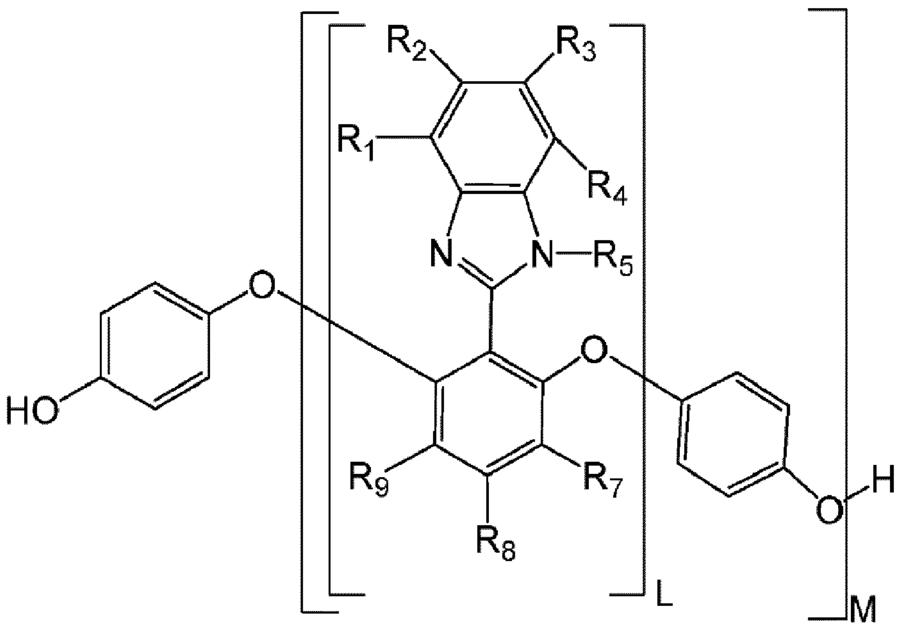

(IVc)

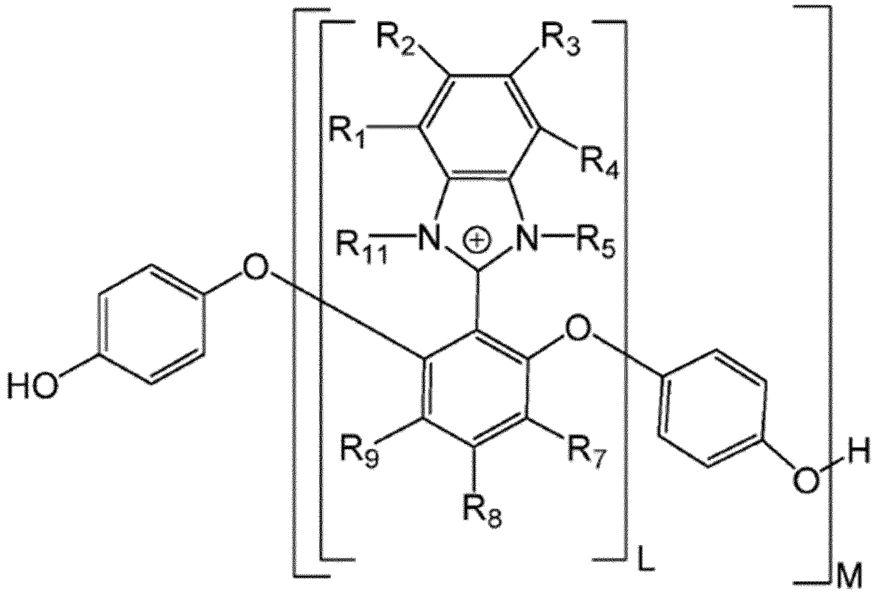

(IVd)

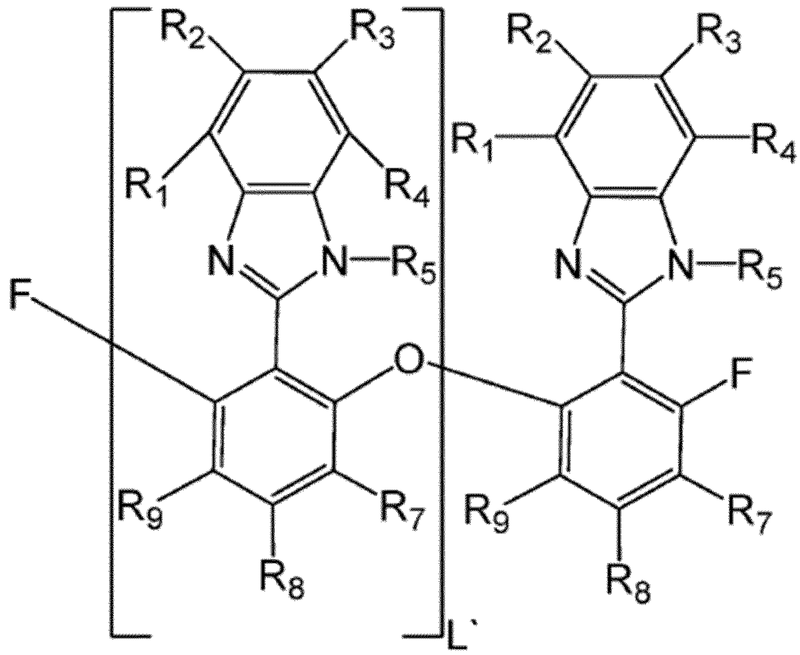

(IVe)

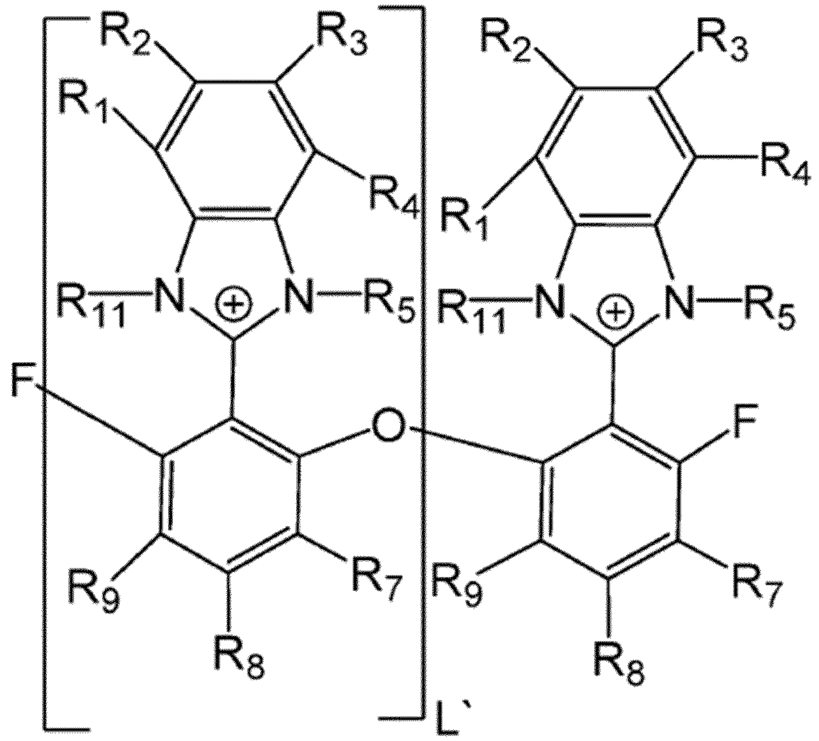

(IVf)

with $R_1$, $R_2$, $R_3$, $R_4$, $R_5$, $R_7$, $R_8$ and $R_9$ as defined in the preceding claims, L = 1 to 25, preferably 1 to 15, more preferably 1 to 9, and L' = 2 to 25, preferably 2 to 15, preferably 3 to 9, and M = 1 to 500.

6. Compound according to any of the preceding claims, **characterized in that** the compound is a compound of the formula (Va) or (Vb)

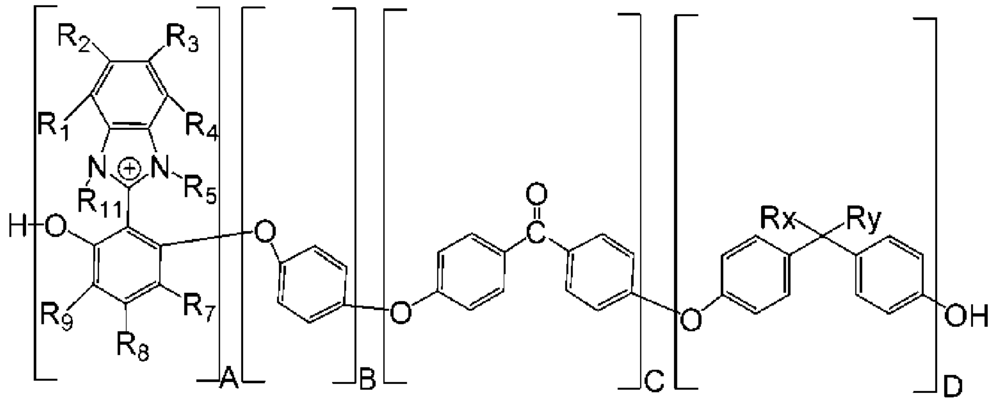

(Va)

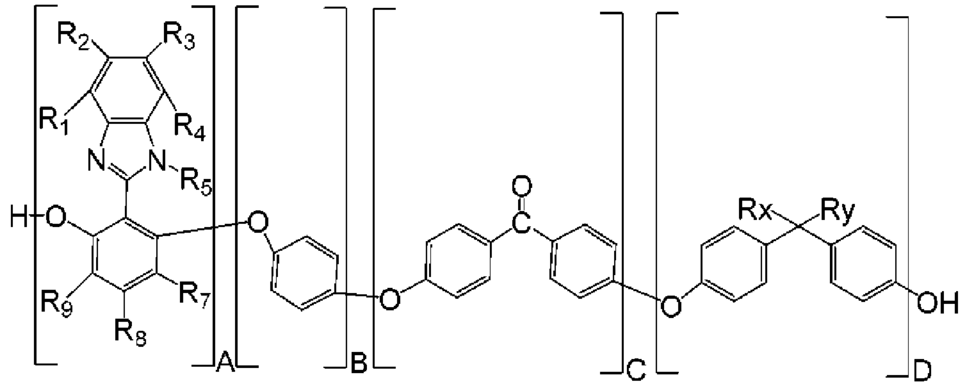

(Vb)

with $R_1$, $R_2$, $R_3$, $R_4$, $R_5$, $R_7$, $R_8$, $R_9$ and $R_{11}$ as defined in the preceding claims and where Rx and Ry are the same or different and are $-CH_3$ or $-CF_3$ and A = 5 to 500, B = 5 to 500, C = 1 to 500 and D = 0 to 1000, where the units indicated by the indices A, B, C and D may occur in blockwise or random distribution in the compound.

7. Compound according to any of the preceding claims, **characterized in that** the compound is a compound of the formula (VIa) to (VId)

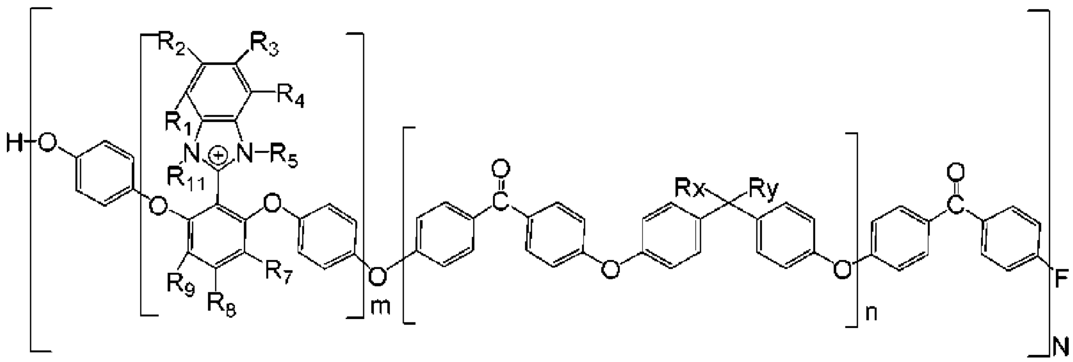

(VIa)

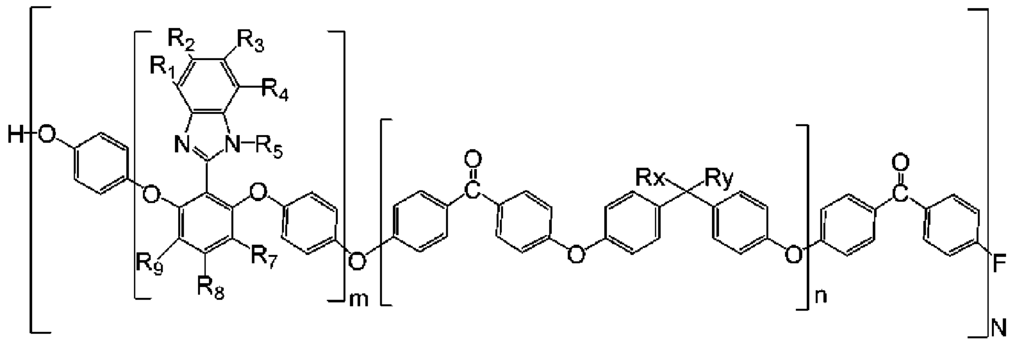

(VIb)

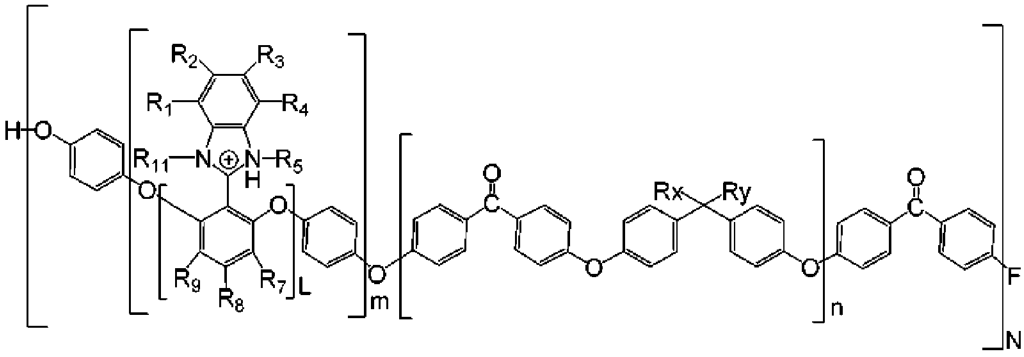

(VIc)

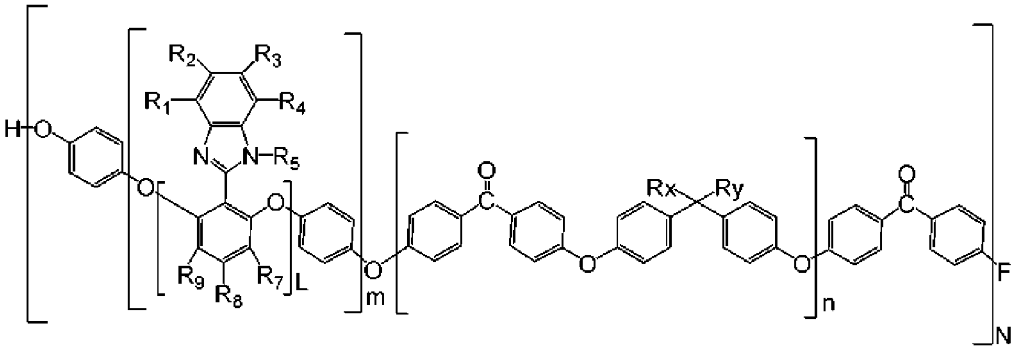

(VId)

with $R_1$, $R_2$, $R_3$, $R_4$, $R_5$, $R_7$, $R_8$, $R_9$ and $R_{11}$ as defined above, L = 1 to 25, preferably 3 to 15, more preferably 3 to 9, and where Rx and Ry are the same or different and are - $CH_3$ or -$CF_3$ and m = 1 to 500, n = 1 to 500, preferably 5 to 50, and N = 1 to 500, where the units indicated by the indices m, n and N may occur in blockwise or random distribution in the compound.

8. Process for preparing compounds according to any of Claims 1 to 7, **characterized in that** it comprises a step in which a compound of the formula (X)

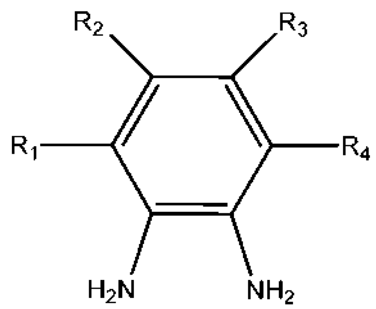

(X)

with $R_1$ to $R_4$ as defined in any of the preceding claims is reacted with a compound of the formula (XI)

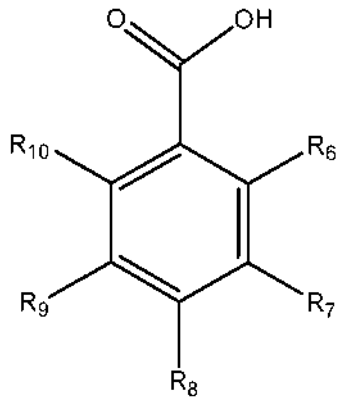

(XI)

with $R_6$ to $R_{10}$ as defined in any of the preceding claims, where $R_6$ and $R_{10}$ = -F, to give a compound (XII)

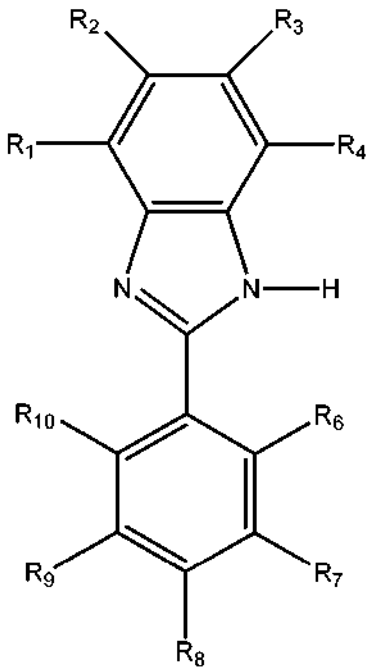

(XII)

and the compound of the formula (XII) is reacted with a methylating reagent or trifluoromethylating reagent to obtain a compound of the formula (Ia) as defined in Claim 1 where $R_5$ = alkyl or perfluoroalkyl radical and $R_6$ and $R_{10}$ = -F.

9. Process according to Claim 8, **characterized in that** it has a process step in which a compound of the formula (Ia) as defined in Claim 1 is reacted with a silane to obtain a compound of the formula (Ia) with $R_{10}$ or $R_6$ = - OH, referred to hereinafter as compound of the formula (Ia') .

10. Process according to Claim 9, **characterized in that** it has a process step in which a compound of the formula (Ia) is reacted with at least one compound of the formula (Ia') to give an oligomer of the formula (Ia").

11. Process according to any of Claims 8 to 10, **characterized in that** it has a process step in which a compound of the formula (Ia) as defined in Claim 1 or formula (Ia") as defined in Claim 11 is reacted with a diol.

12. Process according to any of Claims 8 to 11, **characterized in that** it has a process step in which the reaction product of a compound of the formula (Ia) as defined in Claim 1 or formula (Ia") as defined in Claim 11 with a diol is reacted with a compound of the formula (XIII)

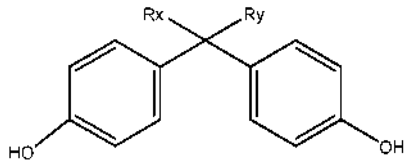

(XIII)

where Rx and Ry are the same or different and are an alkyl or perfluoroalkyl radical, and a difluoro compound or a compound of the formula (XIV)

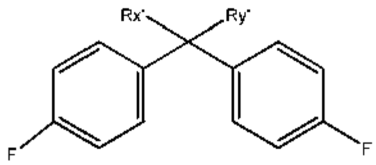

(XIV)

where Rx' and Ry' are the same or different and are an alkyl or perfluoroalkyl radical.

**13.** Process according to Claim 12, **characterized in that** the product obtained in the process step according to Claim 10 is reacted with an alkylating reagent, preferably methylating reagent.

**14.** Use of a polymer according to any of Claims 1 to 7 for production of an anion-conducting membrane.

**15.** Use of a polymer according to any of Claims 1 to 7 as anion-conducting membrane or for production of a component which is used in an electrochemical process, preferably selected from electrolysis, electrodialysis and fuel cell technology.

**16.** Electrolyser, **characterized in that** it includes a polymer according to any of Claims 1 to 7.

**17.** Anion-conducting membrane comprising a polymer according to any of Claims 1 to 7.

**Revendications**

**1.** Composé contenant au moins un élément de formule (Ia) ou (Ib)

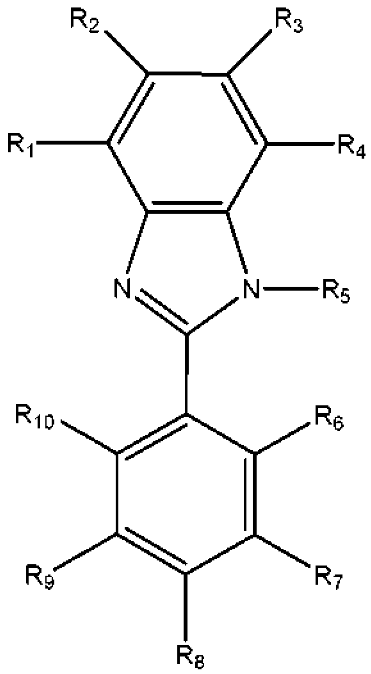

(Ia)

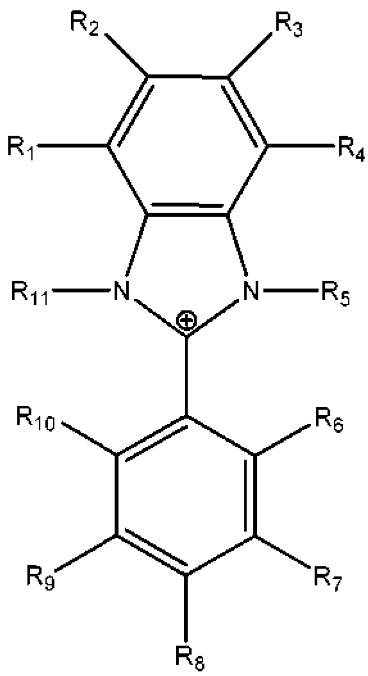

(Ib)

dans lesquelles $R_1$, $R_2$, $R_3$ et $R_4$ représentent, de manière identique ou différente, -H ou un groupe quelconque, de préférence -H,
$R_5$ et $R_{11}$, de manière identique ou différente, représentent un radical alkyle ou perfluoroalkyle,
$R_6$ et $R_{10}$ représentent, de manière identique ou différente, un radical oligomère ou polymère et
$R_7$, $R_8$ et $R_9$ représentent, de manière identique ou différente, -H ou un groupe quelconque, de préférence - H,
**caractérisé en ce qu'**au moins l'un des radicaux $R_6$ ou
$R_{10}$ représente un radical oligomère ou polymère qui est lié à l'atome de carbone de cycle par l'intermédiaire d'un atome d'oxygène.

**2.** Composé selon la revendication 1, **caractérisé en ce que** le composé est un oligomère ou un polymère, qui présente au moins 2 éléments de formule (Ia) ou (Ib), de préférence (Ib).

**3.** Composé selon la revendication 1 ou 2, **caractérisé en ce que** $R_6$ et $R_{10}$ représentent un radical polymère.

**4.** Composé selon l'une des revendications 1 à 3, **caractérisé en ce que** le composé est un composé polymère, dont les radicaux polymères $R_6$ et $R_{10}$ présentent un ou plusieurs des éléments de formule (IIa) et/ou (IIb) et/ou (IIIa) et/ou (IIIb)

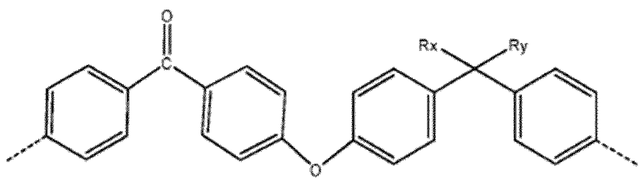

(IIa)

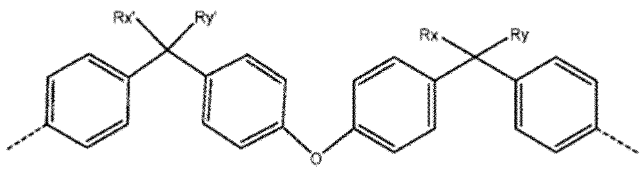

(IIb)

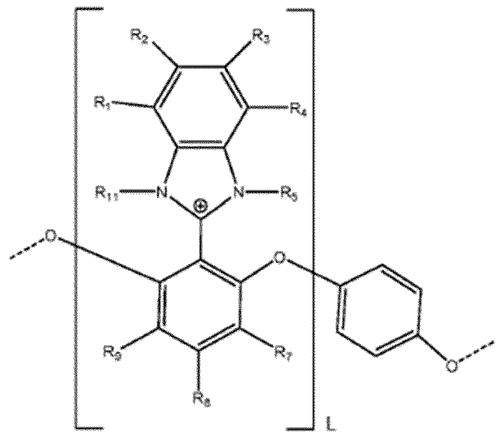

(IIIa)

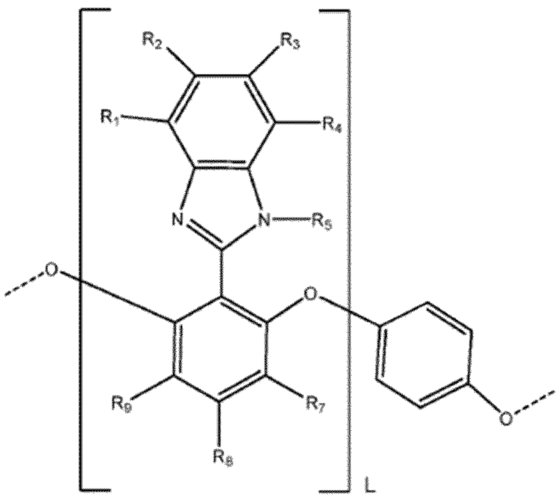

(IIIb)

dans lesquelles $R_1$, $R_2$, $R_3$, $R_4$, $R_5$, $R_7$, $R_8$, $R_9$ et $R_{11}$ sont tels que définis dans les revendications précédentes, L = 1 à 25, de préférence 1 à 15, préférablement 1 à 9 et Rx et Ry et Rx' et Ry' représentent, de manière identique ou différente, un radical alkyle, phényle ou perfluoroalkyle.

**5.** Composé selon l'une des revendications précédentes, **caractérisé en ce que** le composé est un composé de formule (IVa), (IVb), (IVc), (IVd), (IVe) ou (IVf)

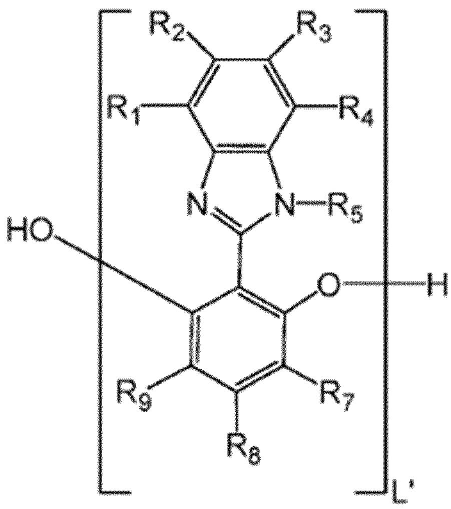

(IVa)

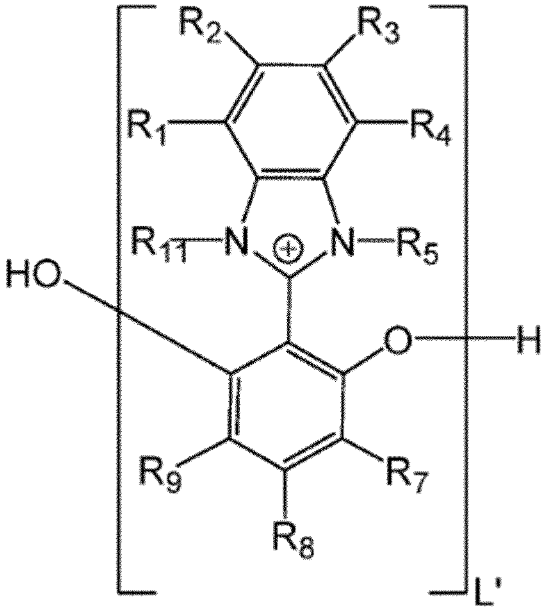

(IVb)

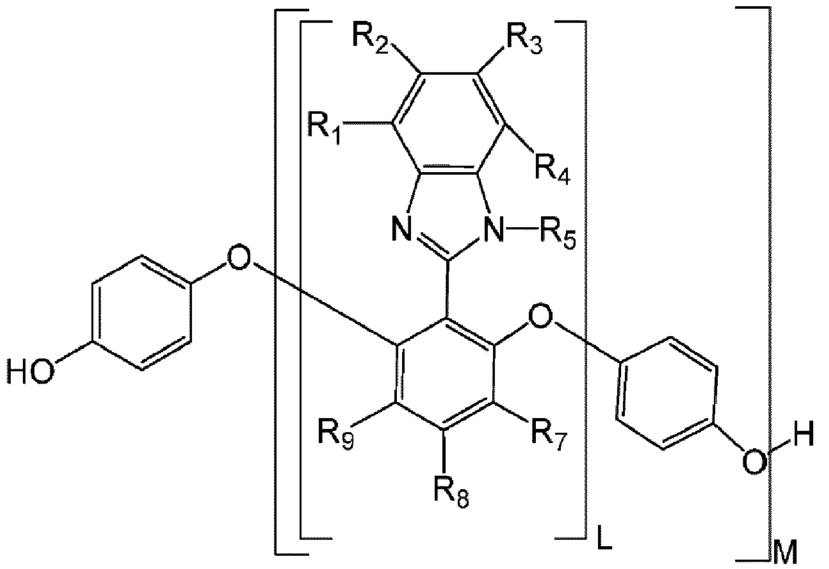

(IVc)

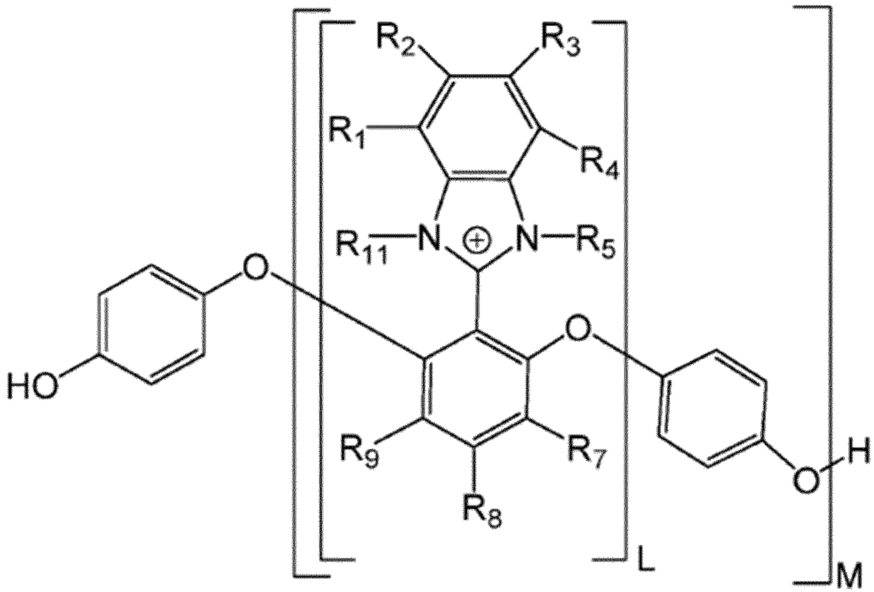

(IVd)

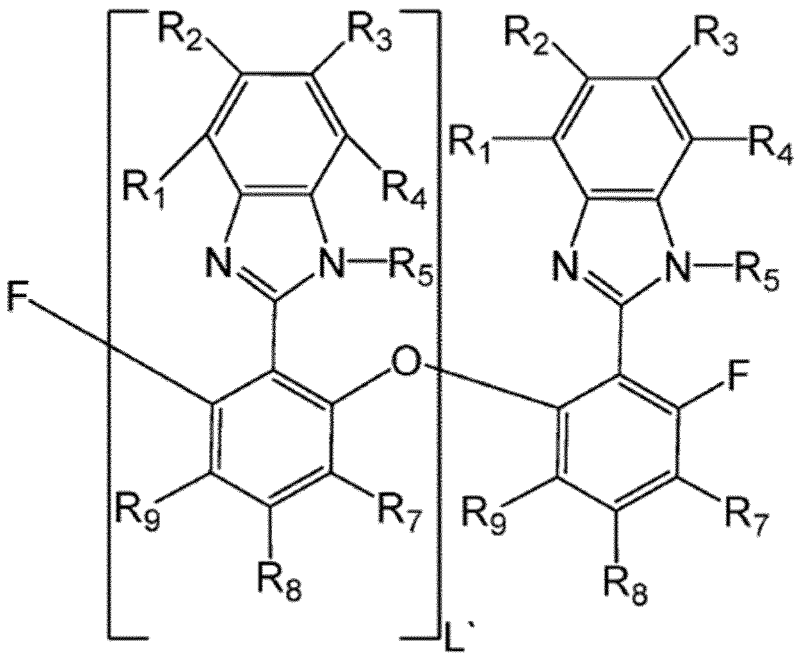

(IVe)

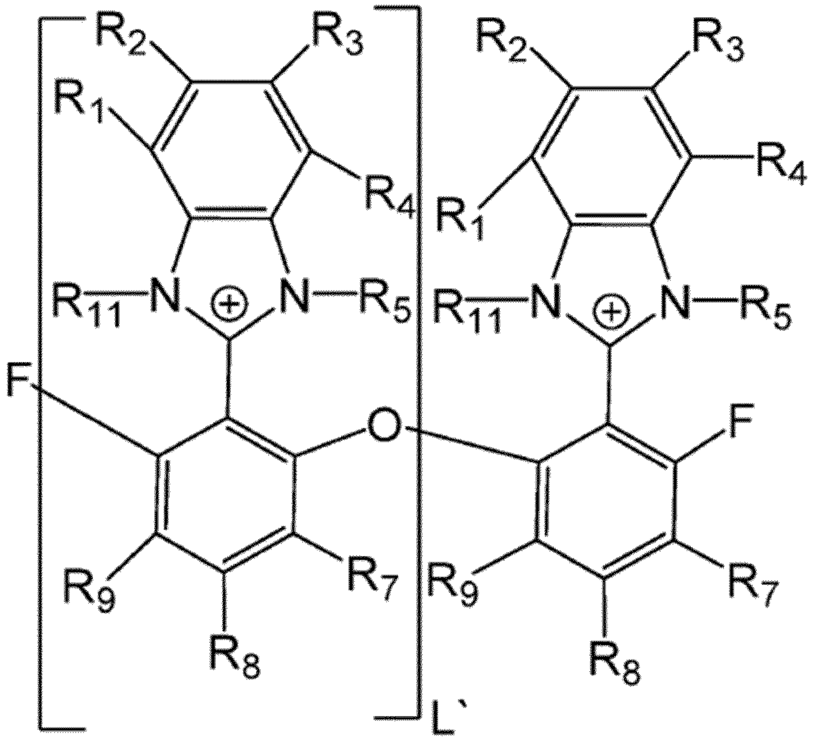

(IVf)

dans lesquelles $R_1$, $R_2$, $R_3$, $R_4$, $R_5$, $R_7$, $R_8$ et $R_9$ sont tels que définis dans les revendications précédentes, L = 1 à 25, de préférence 1 à 15, préférablement 1 à 9 et L' = 2 à 25, de préférence 2 à 15, préférablement 3 à 9 et M = 1 à 500.

**6.** Composé selon l'une des revendications précédentes, **caractérisé en ce que** le composé est un composé de formule (Va) ou (Vb)

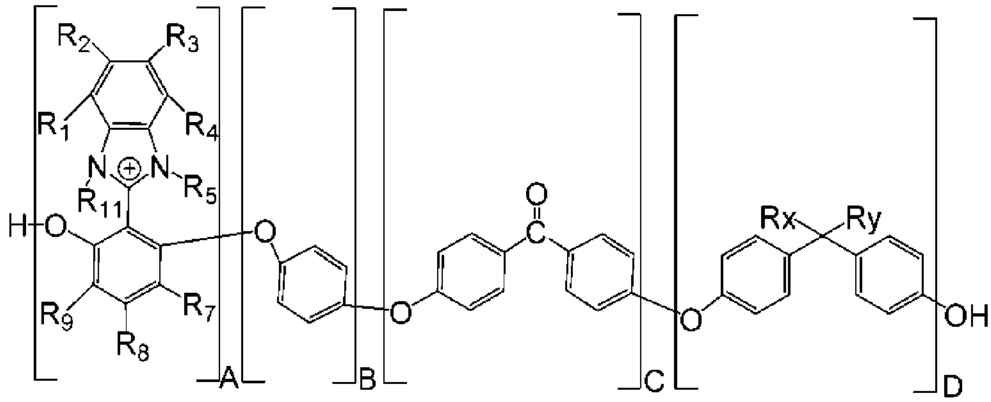

(Va)

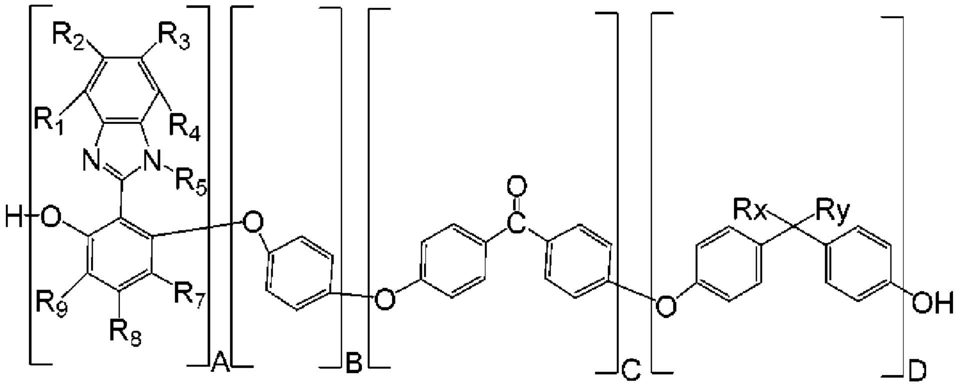

(Vb)

dans lesquelles $R_1$, $R_2$, $R_3$, $R_4$, $R_5$, $R_7$, $R_8$, $R_9$ et $R_{11}$ sont tels que définis dans les revendications précédentes et Rx et Ry représentent, de manière identique ou différente, $-CH_3$ ou $-CF_3$ et A = 5 à 500, B = 5 à 500, C = 1 à 500 et D = 0 à 1000, les motifs **caractérisés par** les indices A, B, C et D pouvant se trouver sous forme de blocs ou sous forme statistiquement répartie dans la composé.

7. Composé selon l'une des revendications précédentes, **caractérisé en ce que** le composé est un composé des formules (VIa) à (VId)

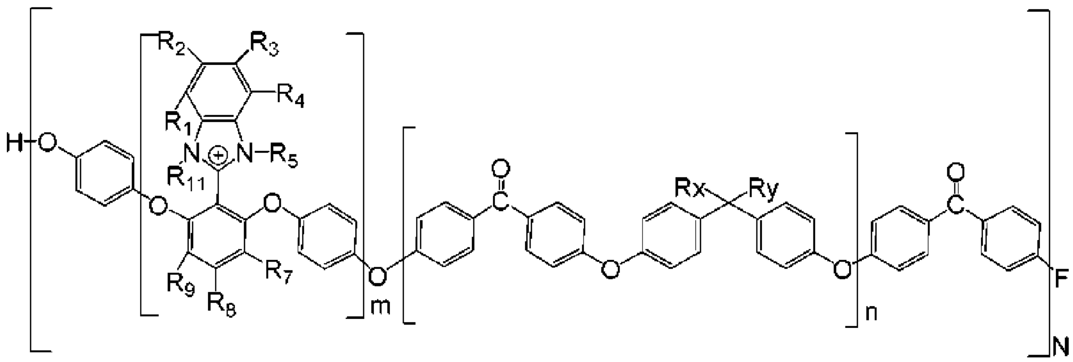

(VIa)

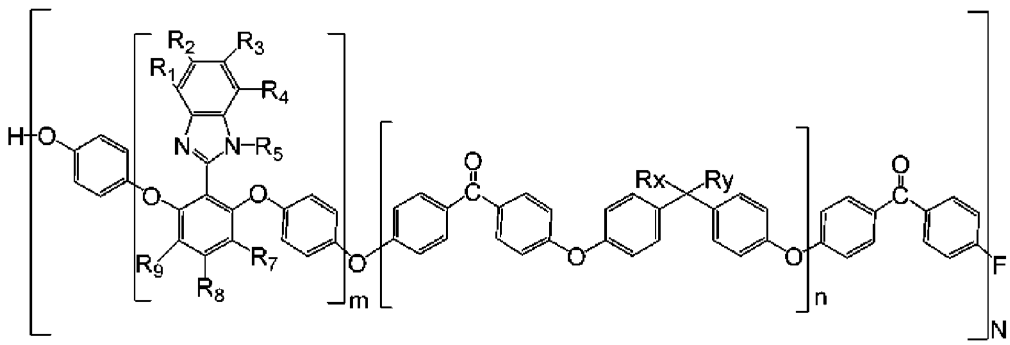

(VIb)

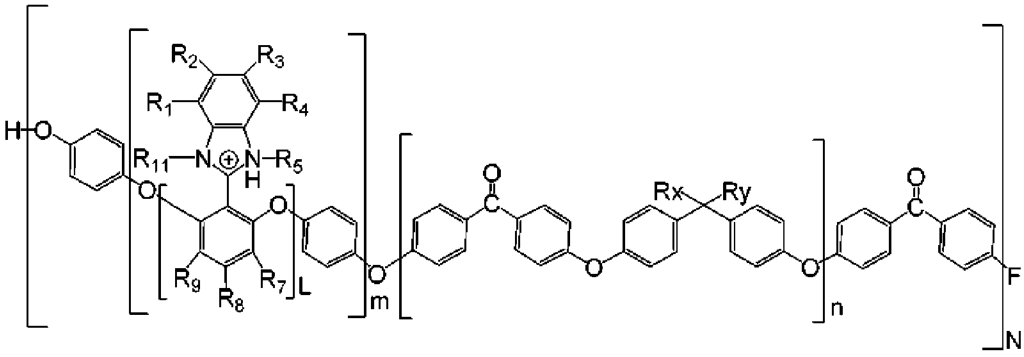

(VIc)

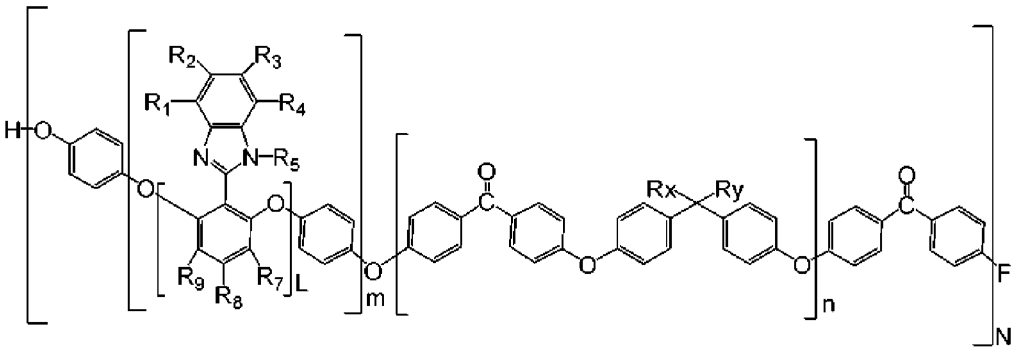

(VId)

dans lesquelles $R_1$, $R_2$, $R_3$, $R_4$, $R_5$, $R_7$, $R_8$, $R_9$ et $R_{11}$ sont tels que définis ci-dessus, L = 1 à 25, de préférence 3 à 15, préférablement 3 à 9, et Rx et Ry représentent, de manière identique ou différente, $-CH_3$ ou $-CF_3$ et m = 1 à 500, n = 1 à 500, de préférence 5 à 50 et N = 1 à 500, les motifs **caractérisés par** les indices m, n et N pouvant se trouver sous forme de blocs ou sous forme statistiquement répartie dans la composé.

**8.** Procédé pour la préparation de composés selon l'une des revendications 1 à 7, **caractérisé en ce qu'**il contient une étape dans laquelle un composé de formule (X)

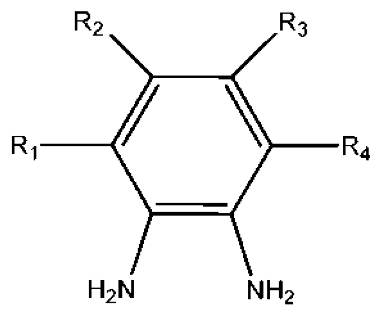

(X)

dans laquelle $R_1$ à $R_4$ sont tels que définis dans les revendications précédentes, est transformé avec un composé de formule (XI)

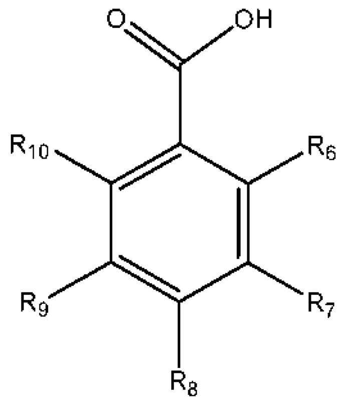

(XI)

dans laquelle $R_6$ à $R_{10}$ sont tels que définis dans les revendications précédentes, $R_6$ et $R_{10}$ = -F, en un composé (XII)

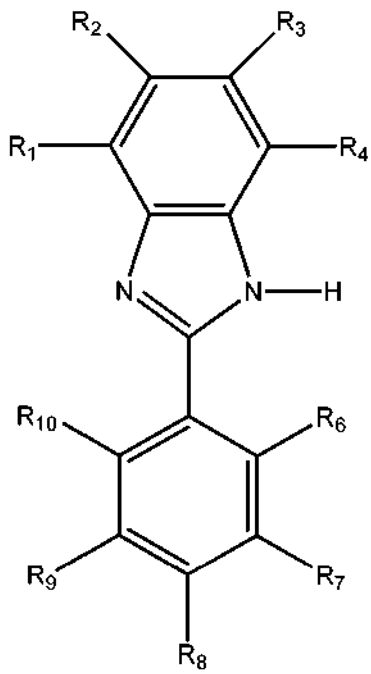

(XII)

,

et le composé de formule (XII) est transformé avec un réactif de méthylation ou un réactif de trifluorométhylation, avec obtention d'un composé de formule (Ia), telle que définie dans la revendication 1, $R_5$ = un radical alkyle ou perfluoroalkyle et $R_6$ et $R_{10}$ = -F.

**9.** Procédé selon la revendication 8, **caractérisé en ce qu'**il présente une étape de procédé dans laquelle un composé de formule (Ia), telle que définie dans la revendication 1, est transformé avec un silane avec obtention d'un composé de formule (Ia) dans laquelle $R_{10}$ ou $R_6$ = -OH, désigné par composé de formule (Ia') dans la suite.

**10.** Procédé selon la revendication 9, **caractérisé en ce qu'**il présente une étape de procédé dans laquelle un composé de formule (Ia) est transformé avec au moins un composé de formule (Ia') en un oligomère de formule (Ia").

**11.** Procédé selon l'une des revendications 8 à 10, **caractérisé en ce qu'**il présente une étape de procédé dans laquelle un composé de formule (Ia), telle que définie dans la revendication 1, ou de formule (Ia"), telle que définie dans la revendication 10, est transformé avec un diol.

**12.** Procédé selon l'une des revendications 8 à 11, **caractérisé en ce qu'**il présente une étape de procédé dans laquelle

le produit de transformation d'un composé de formule (Ia), telle que définie dans la revendication 1, ou de formule (Ia"), telle que définie dans la revendication 10, est transformé avec un diol, avec un composé de formule (XIII)

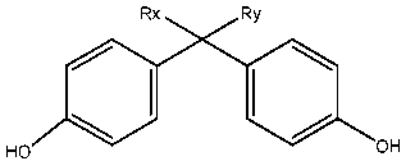

(XIII)

dans laquelle Rx et Ry, de manière identique ou différente, représentent un radical alkyle ou perfluoroalkyle, et un composé bifluoré ou un composé de formule (XIV)

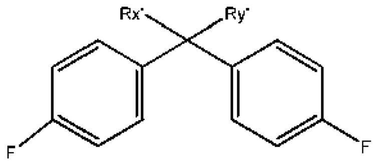

(XIV)

dans laquelle Rx' et Ry', de manière identique ou différente, représentent un radical alkyle ou perfluoroalkyle.

**13.** Procédé selon la revendication 12, **caractérisé en ce que** le produit obtenu dans l'étape de procédé selon la revendication 10 est transformé avec un réactif d'alkylation, de préférence un réactif de méthylation.

**14.** Utilisation d'un polymère selon l'une des revendications 1 à 7 pour la préparation d'une membrane conductrice d'anions.

**15.** Utilisation d'un polymère selon l'une des revendications 1 à 7 comme membrane conductrice d'anion ou pour la préparation d'un composant qui est utilisé dans un procédé électrochimique, de préférence choisi parmi l'électrolyse, l'électrodialyse et la technologie des piles à combustible.

**16.** Électrolyseur, **caractérisé en ce qu'**il présente un polymère selon l'une des revendications 1 à 7.

**17.** Membrane conductrice d'anions, contenant un polymère selon l'une des revendications 1 à 7.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005045978 A2
- US 2009325030 A1
- US 20040121210 A1
- EP 2224523 B1
- US 20140014519 A1
- EP 2296210 A1
- EP 2606954 A1
- WO 2013149328 A
- EP 0126231 B1
- US 3817749 A
- WO 2017117678 A